# EUROPEAN PATENT SPECIFICATION

(11) **EP 2 794 886 B1**
(45) Date of publication and mention of the grant of the patent: **23.11.2016**
(21) Application number: 12821339.4
(22) Date of filing: 12.12.2012
(51) Int. Cl.: C12N 15/80

(54) **IMPROVING FUNGAL GENE EXPRESSION USING INSULATOR DNA SEQUENCES**
VERBESSERTE PILZGENEXPRESSION MIT ISOLATOR-DNA-SEQUENZEN
AMÉLIORATION DE L'EXPRESSION GÉNIQUE FONGIQUE À L'AIDE DE SÉQUENCES D'ADN DU TYPE ISOLATEURS

(30) Priority: 21.12.2011 US 201161578639 P
(43) Date of publication of application: 29.10.2014
(73) Proprietor: Danisco US Inc., Palo Alto, California 94304 (US)
(72) Inventor: BARENDS, Sharief, Palo Alto, CA 94304 (US); WARD, Michael, Palo Alto, CA 94304 (US)
(74) Representative: Kiddle, Simon John
(86) International application number: PCT/US2012/069265
(87) International publication number: WO 2013/096056

(56) References cited:
- EP-A1- 1 887 083
- WO-A1-2004/035780
- WO-A1-2005/040384
- WO-A2-2006/055931
- CN-A- 101 285 072
- JIN XIN ET AL: "Heterologous expression of an endo-beta-1,4-glucanase gene from the anaerobic fungus Orpinomyces PC-2 in Trichoderma reesei", WORLD JOURNAL OF MICROBIOLOGY & BIOTECHNOLOGY, vol. 27, no. 12, 15 May 2011 (2011-05-15), pages 2913-2920, XP002696230,
- YAHATA ET AL: "cHS4 Insulator-mediated Alleviation of Promoter Interference during Cell-based Expression of Tandemly Associated Transgenes", JOURNAL OF MOLECULAR BIOLOGY, ACADEMIC PRESS, UNITED KINGDOM, vol. 374, no. 3, 1 November 2007 (2007-11-01), pages 580-590, XP022324995, ISSN: 0022-2836, DOI: 10.1016/J.JMB.2007.09.054
- XIN BI ET AL: "Chromosomal boundaries in S. cerevisiae", CURRENT OPINION IN GENETICS & DEVELOPMENT, vol. 11, no. 2, 1 April 2001 (2001-04-01), pages 199-204, XP055061303, ISSN: 0959-437X, DOI: 10.1016/S0959-437X(00)00179-9
- WEST A G ET AL: "Insulators: Many functions, many mechanisms", GENES AND DEVELOPMENT, COLD SPRING HARBOR LABORATORY PRESS, PLAINVIEW, NY, US, vol. 16, no. 3, 1 February 2002 (2002-02-01), pages 271-288, XP002249349, ISSN: 0890-9369, DOI: 10.1101/GAD.954702
- DAVID W. EMERY: "The Use of Chromatin Insulators to Improve the Expression and Safety of Integrating Gene Transfer Vectors", HUMAN GENE THERAPY, vol. 22, no. 6, 1 June 2011 (2011-06-01), pages 761-774, XP055061191, ISSN: 1043-0342, DOI: 10.1089/hum.2010.233
- GEYER P K ET AL: "Protecting against promiscuity: The regulatory role of insulators", CMLS CELLULAR AND MOLECULAR LIFE SCIENCES, BIRKHAUSER VERLAG, HEIDELBERG, DE, vol. 59, no. 12, 1 December 2002 (2002-12-01), pages 2112-2127, XP002573586, ISSN: 1420-682X, DOI: 10.1007/S000180200011

## Description

### TECHNICAL FIELD

The present compositions and methods relate to the use of insulator DNA sequences to increase the expression of genes of interest in fungi.

### BACKGROUND

A significant problem associated with the use of fungi as expression hosts for genes of interest is that the levels of protein produced by different transformants can vary substantially. Identifying transformants that produce a high level of a protein of interest sometimes requires screening hundreds, and even thousands of individual transformants, even though the DNA introduced into each cell is ostensibly the same. This appears to be the case for any genomically-integrated, ectopically-expressed gene of interest, and is believed to be caused by the random nature of genomic integration, and the different chromatin structures present at the site of insertion.

Insertion at sites of "open" regions of genomic DNA (*i.e*., euchromatin) is believed to give rise to higher levels of expression, whereas insertion at sites of more "closed" regions of genomic DNA (*i.e*., heterochromatin) is believed to give rise to reduced levels of expression. As a result of this variation in expression, identifying transformants that express high levels of a protein of interest can be labor-intensive and time consuming. The need exists for ways to reduce screening time and improve the odds for stable integration at open regions of DNA.

### SUMMARY

Described are strains and methods relating to improved fungal expression using insulator sequences in *Trichoderma* spp. filamentous fungal cells as set out in the claims.

In one aspect, the present invention provides an expression cassette for expressing a gene of interest in *Trichoderma* spp. cells, the expression cassette being comprised of a contiguous DNA sequence comprising:
a region encoding a gene of interest desired to be expressed;
one or more regulatory sequences sufficient to cause the expression of the gene of interest upon introduction of the expression vector into the *Trichoderma* spp. cells; and
an insulator sequence upstream and/or downstream of the contiguous DNA sequence, wherein the insulator sequence reduces histone binding;
wherein upon introduction of the expression cassette into the *Trichoderma* spp. cells, the presence of the insulator sequence increases the expression of the gene of interest compared to the level of expression obtained using an equivalent expression cassette lacking the insulator sequences. In some embodiments, the one
or more regulatory sequences includes a promoter, and the insulator sequence is placed upstream of the promoter. In some embodiments, the one or more regulatory sequences includes a terminator,
and the insulator sequence is placed downstream of the terminator. In some embodiments, the one or more regulatory sequences include a promoter and a terminator, and a first insulator sequence is placed upstream of the promoter, and a second insulator sequence is placed downstream of the terminator.

In some embodiments, the insulator sequence is a portion of an rRNA gene.

In some embodiments, the insulator sequence has a predicted histone occupancy probability of less than 0.3. In some embodiments, the insulator sequence has a predicted histone occupancy probability of less than 0.2. In some embodiments, the insulator sequence has a predicted histone occupancy probability of less than 0.1.

In another aspect,, the present invention provides an integration cassette for expressing a gene of interest in *Trichoderma* spp. cells, the integration cassette being comprised of a contiguous DNA sequence comprising:
a site of insertion for a gene of interest desired to be expressed; and
an insulator sequence upstream and/or downstream of the site of insertion, wherein the insulator sequence reduces histone binding;
wherein upon introduction of the integration cassette to *Trichoderma* spp. cells and upon introduction of the gene of interest at the site of insertion, the presence of the insulator sequence increases the expression of the gene of interest compared to the level of expression obtained using an equivalent expression cassette lacking the insulator sequences.

In some embodiments, the integration cassette further comprises a promoter, and the insulator sequence is placed upstream of the promoter. In some embodiments, the integration cassette further comprises a terminator, and the insulator sequence is placed downstream of the terminator. In some embodiments, the integration cassette further comprises a promoter and a terminator, and a first insulator sequence is placed upstream of the promoter, and a second insulator sequence is placed downstream of the terminator.

In some embodiments, the insulator sequence is a portion of an rRNA gene.

In some embodiments, the insulator sequence has a predicted histone occupancy probability of less than 0.3. In some embodiments, the insulator sequence has a predicted histone occupancy probability of less than 0.2. In some embodiments, the insulator sequence has a predicted histone occupancy probability of less than 0.1.

In another aspect, the present invention provides a method for expressing a gene of interest in *Trichoderma* spp. cells, the method comprising, introducing to the *Trichoderma* spp. cells
(i) a contiguous DNA sequence comprising a region encoding a gene of interest desired to be expressed and one or more regulatory sequences sufficient to cause the expression of the gene of interest in the *Trichoderma* spp. cells; and
(ii) an insulator sequence upstream or downstream of the contiguous DNA sequence in (i), wherein the insulator sequence reduces histone binding;
wherein the presence of the insulator sequence increases the expression of the gene of interest in the *Trichoderma* spp. cells compared to the level of expression obtained in the absence of the insulator sequences.

In some embodiments, the contiguous DNA sequence and insulator sequence are introduced simultaneously into the Trichoderma spp. cells. In some embodiments, the contiguous DNA sequence and insulator sequence are introduced sequentially into the Trichoderma spp. cells. In some embodiments, the gene of interest and regulatory sequences are introduced simultaneously into the Trichoderma spp.. cells as a contiguous DNA sequence. In some embodiments, the gene of interest and regulatory sequences are in an expression cassette. In some embodiments, the gene of interest and regulatory sequences are introduced sequentially into the Trichoderma spp. cells to form a contiguous DNA sequence.

In some embodiments, the regulatory sequences comprises a promoter, and the insulator sequence is placed upstream of the promoter. In some embodiments, the regulatory sequences comprises a terminator, and the insulator sequence is placed downstream of the terminator. In some embodiments, the regulatory sequences comprise a promoter and a terminator, and a first insulator sequence is placed upstream of the promoter, and a second insulator sequence is placed downstream of the terminator.

In some embodiments, the insulator sequence is a portion of an rRNA gene.

In some embodiments, the insulator sequence has a predicted histone occupancy probability of less than 0.3. In some embodiments, the insulator sequence has a predicted histone occupancy probability of less than 0.2. In some embodiments, the insulator sequence has a predicted histone occupancy probability of less than 0.1.

In another aspect, a gene of interest expressed by any of the foregoing methods is provided.

These and other aspects and embodiments of present strains and methods will be apparent from the description, including the accompanying Figures.

### BRIEF DESCRIPTION OF THE DRAWINGS

Figure 1A is a diagram showing DNA wrapped around a histone. Figure 1B shows the predicted probability of histone occupancy for a selected ~300 bp region of DNA.
Figure 2 is a graph showing the effect of upstream and downstream insulator sequences on the expression of a reporter protein from transformants grown in 24-well plates.
Figure 3A is a graph showing the effect of insulator sequences on the mean and median expression levels of a reporter protein, calculated from the data shown in Figure 1B. Figure 3B shows the raw data.
Figures 4A and 4B are Coomassie-stained SDS-PAGE gels showing the protein patterns produced by the highest level-expressing transformants from Figure 2.
Figure 5 is a graph showing the effect of upstream and downstream insulator sequences on the expression of a reporter protein from transformants grown in shake flasks. The transformants are the same as those shown in Figure 4.
Figures 6A and 6B are Coomassie-stained SDS-PAGE gels showing the protein patterns produced by the transformants from Figure 5.
Figure 7 is a graph showing the effect of downstream insulator sequences on the expression of a reporter protein from a predetermined locus.
Figures 8A and 8B are graphs showing the effect of downstream insulator sequences on the expression of a reporter protein from a predetermined locus in transformants grown in 24-well plates (8A) or shake flasks (8B). Figure 8C is a Coomassie-stained SDS-PAGE gel showing the protein patterns produced by the transformants from Figure 8B.

### DETAILED DESCRIPTION

### I. Overview

The present compositions and method are based on the inclusion of certain DNA sequences, associated with naturally "open" regions of genomic DNA (herein referred to as "insulators"), in expression cassettes or integration cassettes, to increase the probability that, once integrated, genes of interest are in a local chromatin context that promotes high levels of expression. While the existence of such DNA sequences in the genomic DNA of some eukaryotic organisms has been described, the inclusion of such sequences in expression cassettes or integration cassettes to facilitate increased ectopic gene expression in fungi is heretofore unknown and offers significant advantages over conventional expression vectors.

### II. Definitions

Prior to describing the present compositions and methods in detail, the following terms are defined for clarity. Terms not defined should be accorded their ordinary meanings as used in the relevant art.

As used herein, "nucleosomes" refer to the basic repeating structures of chromatin. Formally, a nucleosome is accepted to include a 147-bp stretch of DNA sharply bent around a histone octamer, which includes several different histone polypeptides. For the purposes of the present compositions and methods, nucleosomes are sufficiently defined as regions of DNA bent around a "histone," referring collectively to the various discrete histone polypeptides.

As used herein, an "expression cassette" refers to the coding sequence of a protein of interest [herein also referred to as a gene of interest (see, below)], operably linked to a minimal set of regulatory sequences that are sufficient to direct expression of the protein of interest in fungi. Generally, the minimal regulatory sequences include a promoter and terminator. Regulatory sequences include optional enhancers, repressors, introns, or other *cis*-acting sequences, intended to regulate the expression of the protein of interest. An expression cassette is generally part of an expression vector, which may further include a selectable marker, sequences to direct genomic integration *(i.e.,* recombination sequences), an origin of replication, and the like. Although the compositions and methods are primarily described with respect to a "protein" of interest, they also apply to the expression of rRNAs of interest, hence the indiscriminate use of the term gene of interest (see, below).

As used herein, an "integration cassette" refers to a DNA sequence for introduction into the genome of fungi for the purpose of promoting the later integration of a gene of interest. The integration cassette may include recombination sequence suitable for promoting integration of the gene of interest. The gene of interest may be in the form of an expression cassette, as described, above. Alternatively, the integration cassette may include one or more regulatory sequences for promoting the expression of the gene of interest, in which case such regulatory need not be included in the expression cassette. The integration cassette is generally part of a vector, which may further include a selectable marker, sequences to direct genomic integration (*i.e*., recombination sequences), an origin of replication, and the like.

As used herein, an "open region of genomic DNA" and similar terms, refer to regions of genomic DNA that are relatively nucleosome-free and methylation-free, such that they are relatively accessible to transcription factors. Such regions are associated with the term "euchromatin."

As used herein, a "closed region of genomic DNA" and similar terms, refer to regions of genomic DNA that are associated with a relatively large number of nucleosomes, and may be methylated, such that they are relatively inaccessible to transcription factors. Such regions are associated with the term "heterochromatin."

As used herein, an "insulated expression cassette" is an expression cassette that includes at least one insulator DNA sequence either upstream of a promoter or downstream of a terminator sequence.

As used herein, the term "upstream" means 5' with respect to a reference sequence, such a sequence encoding a gene of interest, a promoter, a terminator, and the like.

As used herein, the term "downstream" means 3' with respect to a reference sequence, such a sequence encoding a gene of interest, a promoter, a terminator, and the like.

As used herein, "introducing" a DNA sequence into a cell refers to transformation and genomic integration of the DNA sequence. Simultaneous introduction refers to a single transformation and integration event, while sequential introduction refers to a plurality of independent transformation and integration events.

As used herein, "insulator DNA sequences," insulator sequences," and "insulators," refer to portions of DNA that are relatively nucleosome-free when present in the genomic DNA of filamentous fungi. Examples are provided herein. Insulators need not be derived from filamentous fungus genomic DNA but must function as insulators in filamentous fungi.

As used herein, "filamentous fungi" are members of the Basidiomycota or the Ascomycota. Of particular interest are members of the phylum Ascomycota, subphylum Pezizomycotina. Examples of filamentous fungus are provided, herein.

As used herein, "*Trichoderma reesei*" refers to a particular filamentous fungus previously classified as *Trichoderma longibrachiatum*, and also as *Hypocrea jecorina*.

As used herein, the term "gene of interest" refers to a gene product that is desired to be expressed in a filamentous fungus, optionally at high levels, and for the purpose of commercialization. Such a gene product may be an enzyme, a substrate-binding protein, a surface-active protein, a structural protein, an rRNA, or the like. The term "protein of interest" is used without distinction but the skilled reader will appreciate that the compositions and methods can be used for the production of rRNA. Genes, protein, and rRNAs of interest are to be distinguished from myriad other products expressed by the filamentous fungus cells, which are generally not of interest as products, and are mainly considered background contaminants.

As used herein, the terms "polypeptide" and "protein" are used interchangeably to refer to polymers of any length comprising amino acid residues linked by peptide bonds. The conventional one-letter or three-letter codes for amino acid residues are used herein. Polypeptides may include disulfide bonds, glycosylation, lipidation, acetylation, phosphorylation, or any other modifications.

As used herein, the term "gene" is synonymous with the term "allele" in referring to a nucleic acid that encodes and directs the expression of a protein or rRNA. Vegetative forms of filamentous fungi are generally haploid, therefore a single copy of a specified gene *(i.e.,* a single allele) is sufficient to confer a specified phenotype.

As used herein, a "contiguous DNA sequence" is a DNA sequence that includes specified elements, such as a coding sequence, regulatory elements, insulators, and the like, which are substantially uninterrupted by unspecified elements.

As used herein, the term "cell broth" refers collectively to medium and cells in a liquid/submerged culture.

As used herein, the term "cell mass" refers to the cell component (including intact and lysed cells) present in a liquid/submerged culture. Cell mass may be expressed in dry or wet weight.

As used herein, the singular articles "a," "an," and "the" encompass the plural referents unless the context clearly dictates otherwise. The following abbreviations/acronyms have the following meanings unless otherwise specified:
- bp: base pairs
- kb: kilobase
- MW: molecular weight
- w/v: weight/volume
- w/w: weight/weight
- v/v: volume/volume
- wt%: weight percent
- °C: degrees Centigrade
- H₂O: water
- dH₂O or DI: deionized water
- dIH₂O: deionized water, Milli-Q filtration
- g or gm: gram
- µg: microgram
- mg: milligram
- kg: kilogram
- lb: pound
- µL and µl: microliter
- mL and ml: milliliter
- mm: millimeter
- µm: micrometer
- M: molar
- mM: millimolar
- µM: micromolar
- U: unit
- sec: second
- min: minute
- hr: hour
- EtOH: ethanol
- NaOAc: Sodium acetate
- BSA: Bovine serum albumin
- eq.: equivalent
- N: normal
- PCR: polymerase chain reaction
- DNA: deoxyribonucleic acid
- FOA: fluoroorotic acid
- rpm: revolutions per minute
- YEG: yeast extract + glucose medium
- rRNA: ribosomal RNA
- ∼: approximately

### III. Identification of insulators in filamentous fungi

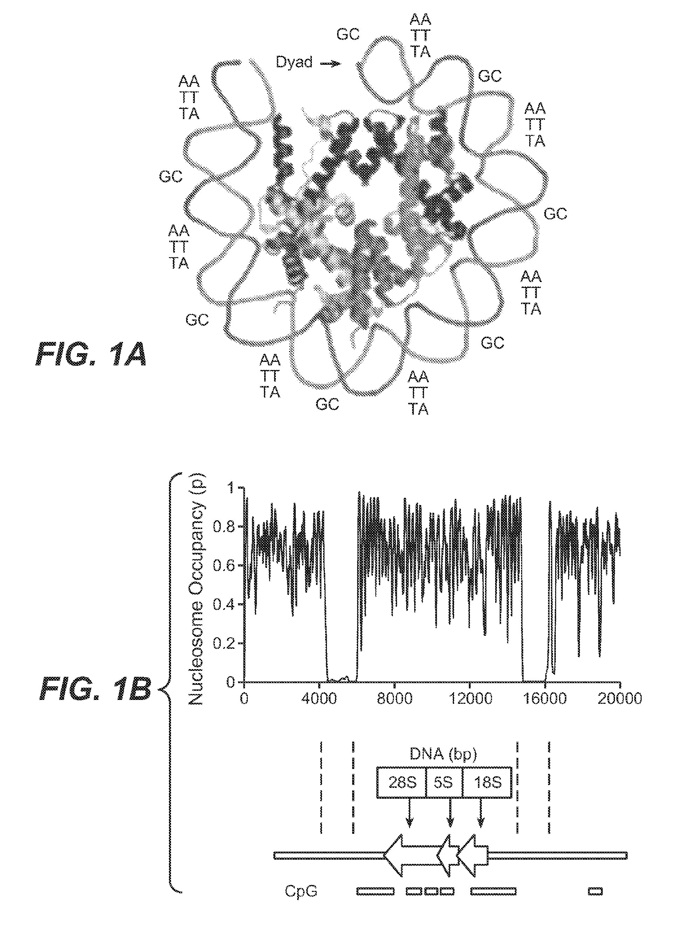

Insulator DNA sequences, or "insulators," refer to portions of nucleosome-free DNA, which are generally more "open" to cellular transcription machinery than DNA that is associated with nucleosomes. Nucleosomes are the basic repeating structures of chromatin, which include a stretch of DNA (generally accepted to be 147-bp) that is sharply bent around a histone octamer (herein referred to collectively as "a histone"). Nucleosomes are separated by linker DNA (usually 10-50-bp in length), which is not associated with histones. Certain DNA sequences reduce the energetic cost of the sharp bending around histones, thereby increasing their overall stability (see, *e.g.,* Segal, E. et al. (2006) Nature 442:772-78). These sequences reduce the energetic cost of sharply bending around a nucleosome. This energy is the major contributor to the final stability of a nucleosome. Each nucleosome contains a 147-bp stretch of DNA, which is sharply bent and tightly wrapped around a histone protein octamer. This sharp bending occurs at every DNA helical repeat (~10 bp), when the major groove of the DNA faces inwards towards the histone octamer, and again ~5 bp away, with opposite direction, when the major groove faces outward. Bends of each direction are facilitated by specific dinucleotides with a 10-bp periodicity, *i.e.,* AA/TT/TA and out of phase GC (see Figure 1A). DNA bending is dominated by a structural deformation known as basepair roll (a rotation of basepair about its long axis, relative to the preceding basepair). AA/TT/TA nucleotides are preferred over others for bending via either a negative or positive basepair roll, whereas GC dinucleotides favor the opposite base-pair roll.

Based on studies with some eukaryotic cells, one would expect to find nucleosome-free DNA associated at least with non-coding (*i.e*., regulatory) regions of highly expressed genes. However, non-coding regions generally have a low degree of conservation among species, making is difficult to identify insulator sequences. Moreover, insulator sequences have not been described in fungi, nor has a significant amount of work been done on fungal chromatin structure.

The approach used in support of the present compositions and methods was to identify probable, highly-expressed genes among different species of fungi (in this case, the filamentous fungi *Trichoderma reesei, T. virens*, and *T. atroviride*), and manually inspect the non-coding regions (excluding promoters) for probability of nucleosome binding using a web-based sequence analyzer. Since rRNA genes and telomeres are generally free of nucleosomes, these regions were selected as starting points. Two putative insulator sequences were identified in an rRNA locus, one of which was selected for study (see Figure 1B). As demonstrated in the appended Examples, the presence of an insulator sequence in an expression cassette, whether upstream, downstream, or both upstream and downstream of a gene of interest significantly increased ectopic expression of the gene in fungi.

### IV. Insulators as components of expression cassettes

In one aspect, the present compositions and methods are based on the inclusion of an insulator sequence as a component of an expression cassette, to ensure that, once integrated, the "insulated" expression cassette is situated in a locally open region of chromatin. In essence, such an expression cassette defines its own context, insulating the gene of interest from the effects of chromatin structure at its site of integration. In this manner, the insulator sequence prevents genomic heterochromatin from engulfing the expression cassette. Insulated expression cassettes increase the mean and median levels of expression from any group of transformants, and greatly reduce the amount of screening necessary to obtain high expression transformants for use as production strains.

Insulators for use according to the present compositions and methods include DNA sequence known to produce localized "open" chromatin structure, typically by interfering with normal histone binding. Such DNA sequences often have continuous stretches of AT-rich sequences. Functionally, insulating DNA sequences are those that do not result in a reduced energy cost for the DNA sequence bending around a nucleosome, compared to a random DNA sequence. In other words, insulator sequences impart an increased energy cost for the DNA sequence bending around a nucleosome, compared to a random DNA sequence. Structurally, insulating sequences deviate from a 10-bp periodicity of AA, TT or TA out of phase (by 5 bp) with a 10-bp periodicity of GC. Examples include, but are not limited to, a sequence of A residues, G residues, C residues, T residues, or random combinations of A and T residues, A and C residues, A and G residues, C and T residues, or G and T residues. Examples even include random combinations of G and C residues that do not induce CpG methylation. Examples also include random combinations of A, C and G residues, A, C and T residues, A, G and T residues, C, G and T residues, or random combinations of any nucleotide provided that the sequence does induce CpG methylation, and provided that the insulating sequence deviates from the consensus having a 10-bp periodicity of AA, TT or TA out of phase (by 5 bp) with a 10-bp periodicity of GC. Insulating DNA sequences may also contain (tandem) repeating DNA sequences, or (tandem) inverted repeats of DNA sequences. Typically, insulating sequences share low homologies between different organisms and even between organisms of the same family. However, insulating sequences may reside in genomic contexts that limit genomic variation of the insulating sequences between different species from the same phylum. Insulating sequences may also be associated with expression of genes that are less prone to evolutionary sequence variation. Insulating sequences may be derived from different organisms. Alternatively, insulating DNA sequences may be engineered *in silico* and synthesized. Also, insulating DNA sequences may be hybrid forms of natural occurring insulating sequences and synthetic insulating DNA sequences. Also, insulating DNA sequences may be concatamerized forms of either naturally occurring insulating DNA sequences, synthetic insulating DNA sequences, or hybrid forms of natural occurring insulating sequences and synthetic DNA. Concatamerization can be performed using techniques known to people skilled in the art. The length of a natural endogenous insulating sequence may vary. The minimal length of an insulating DNA sequence is half a nucleosome wrap, *i.e.,* 74 bp. Insulators may be of fungal origin but other eukaryotic insulator sequences are expected to produce a similar effect.

Segal et al. [(2006) Nature 442:772-78)] describe a computational model for the sequence-based prediction of nucleosome positioning in *S. cerevisiae.* This model can be used to identify sequences useful as insulators. The model predicts nucleosome positioning in terms of probability, *i.e.,* from 0 to 1, where 0 is the lowest probability that a particular sequence will be occupied by histones and 1 is the highest probability that a particular sequence will be occupied by histones. In some embodiments, the sequences selected for use as insulators have a probability of occupancy of 0.30 or less, 0.25 or less, 0.20 or less, 0.15 or less, or even 0.10 or less. In some embodiments, the sequences selected for use as insulators have a probability of occupancy that is less than the average probability of occupancy of the host cell genome by an amount of 0.20 or more, 0.25 or more, 0.30 or more, 0.35 or more, or even 0.40 or more. In some embodiments, the sequences selected for use as insulators have a probability of occupancy that is less than the average probability of occupancy of the surrounding genomic DNA by an amount of 0.20 or more, 0.25 or more, 0.30 or more, 0.35 or more, or even 0.40 or more.

In some embodiments, insulators are placed upstream of (*i.e*., 5'-relative to) a gene of interest in an expression cassette, which generally mean upstream of a promoter operably-linked to a gene of interest. In some embodiments, insulators are placed downstream of (*i.e.,* 3'-relative to) a gene of interest in an expression cassette, which generally means downstream of a terminator sequence operably-linked to a gene of interest. In some embodiments, insulators are placed both upstream and downstream of (*i*.*e*., 5' and 3'-relative to) a gene of interest, as described. Indeed, the effect of placing insulators 5' and 3' of the expression cassette is essentially additive with respect to placing an insulator only on the 5' or 3' side of the cassette.

Where the insulator sequence is located upstream of a promoter, it is preferably placed within 1,000 bp, within 900 bp, within 800 bp, within 700 bp, within 600 bp, within 500 bp, within 400 bp, within 300 bp, within 200 bp, within 100 bp, within 90 bp, within 80 bp, within 70 bp, within 60 bp, within 50 bp, within 40 bp, within 30 bp, within 20 bp, within 10 bp, or even adjacent to the promoter, to minimize the opportunity for the promoter to become part of a nucleosome. The insulator sequence can even overlap with the promoter, assuming that the sequences are compatible and promoter function is not significantly affected.

Where the insulator sequence is located downstream of a terminator, it is preferably placed within 1,000 bp, within 900 bp, within 800 bp, within 700 bp, within 600 bp, within 500 bp, within 400 bp, within 300 bp, within 200 bp, within 100 bp, within 90 bp, within 80 bp, within 70 bp, within 60 bp, within 50 bp, within 40 bp, within 30 bp, within 20 bp, within 10 bp, or even adjacent to the terminator, to minimize the opportunity for the terminator to become part of a nucleosome. The insulator sequence can even overlap with the terminator, assuming that the sequences are compatible and terminator function is not significantly affected.

Insulator sequences may also be placed in one or more introns to reduce the interaction of adjacent exon sequences of a gene of interest with histones.

### V. Insulators as components of integration cassettes

In another aspect, the present compositions and methods are based on the inclusion of an insulator sequence as a component of an integration cassette, which is a DNA sequence that is introduced and stably integrated into a cell to facilitate the future integration of an expression cassette. In this case, the expression cassette may or may not include insulator sequences, since insulator sequences will be provided by the integration cassette. Similar to the above-described insulated expression cassette, an insulated integration cassette defines its own context but with the intention of subsequently providing an insulated context for an expression cassette to be introduced later into the genome.

Insulators for use according to this aspect of the present compositions and methods include those DNA sequences described in the previous section. In some embodiments, insulators are placed upstream of (*i.e*., 5'-relative to) the intended site of integration of an expression cassette. In some embodiments, insulators are placed downstream of (*i.e*., 3'-relative to) the intended site of integration of an expression cassette. In some embodiments, insulators are placed both upstream and downstream of (*i.e*., 5' and 3'-relative to) the intended site of integration of an expression cassette.

As above, the insulator sequence should be located within 1,000 bp, within 900 bp, within 800 bp, within 700 bp, within 600 bp, within 500 bp, within 400 bp, within 300 bp, within 200 bp, within 100 bp, within 90 bp, within 80 bp, within 70 bp, within 60 bp, within 50 bp, within 40 bp, within 30 bp, within 20 bp, within 10 bp, or even adjacent to the site of integration for the expression cassette promoter, to minimize the opportunity for the expression cassette to become part of a nucleosome. The insulator sequence can even overlap with the site of integration, assuming that the sequences are compatible and integration is not significantly affected.

Exemplary integration sites include loci at which highly expressed native genes are known to be located. Notably, the integration cassette can itself be targeted to a preselected site of integration.

Insulator sequences may optionally be present in the expression cassette, as described, thereby further reducing the opportunity for the expression cassette to become part of a nucleosome.

### VI. Utility

The compositions and methods are also contemplated for use with a variety of different host cells. In some aspects of the isclosure, the host cells are fungal cells, including yeast and filamentous fungus cells. Examples of yeast include but are not limited to, *Saccharomyces* spp., *Schizosaccharomyces spp., Arxula* spp., *Candida* spp., *Hansenula* spp., *Kluyveromyces* spp., *Pichia* spp., or a *Yarrowia* spp. Examples of filamentous fungi include, the phyla Basidiomycota and Ascomycota, including the subphylum Pezizomycotina. Exemplary filamentous fungi include, but are not limited, to *Trichoderma* spp., *Aspergillus* spp., *Fusarium* spp., *Scedosporium* spp., *Perticillium* spp., *Chrysosporium* spp., *Cephalosporium* spp., *Talaromyces* spp., *Geosmithia* spp., *Myceliophthora* spp., and *Neurospora* spp. Particular organisms include, but are not limited to, *Trichoderma reesei* (previously classified as *Trichoderma longibrachiatum* and *Hypocrea jecorina*), *Trichoderma virens*, *Trichoderma atroviride, Aspergillus niger, Aspergillus fumigatus, Aspergillus itaconicus, Aspergillus oryzae, Aspergillus nidulans*, *Aspergillus terreus, Aspergillus sojae, Aspergillus japonicus, Scedosporium prolificans*, *Neurospora crassa, Penicillium funiculosum, Penicillium chrysogenum*, *Talaromyces (Geosmithia) emersonii*, *Fusarium vertertatum, Myceliophthora thermophila,* and *Chrysosporium lucknowense*.

The present compositions and methods can also be used with a variety of proteins of interest, which generally include any protein that can be expressed in fungi. Examples of such proteins are amylases, glucoamylases, cellulases, hemi-cellulases, catalases, laccases, proteases, and the like. The increased level of expression made possible by the present insulated expression cassettes and insulated integration cassettes may further allow the production of yet additional proteins of interest in fungi, for example, certain bacterial proteins.

Transformation of insulated expression cassettes and insulated integration cassettes into fungi may be performed by all known methods, including chemical methods, electroporation, and biolistic transformation.

Aspects and embodiments of the present compositions and methods are further described in the following numbered paragraphs:
1. In one aspect, an expression cassette for expressing a gene of interest in fungal cells is provided, the expression cassette being comprised of a contiguous DNA sequence comprising:
   a region encoding a gene of interest desired to be expressed;
   one or more regulatory sequences sufficient to cause the expression of the gene of interest upon introduction of the expression vector into the fungal cells; and
   an insulator sequence upstream and/or downstream of the contiguous DNA sequence, wherein the insulator sequence reduces histone binding;
   wherein upon introduction of the expression cassette into the fungal cells, the presence of the insulator sequence increases the expression of the gene of interest compared to the level of expression obtained using an equivalent expression cassette lacking the insulator sequences.
2. In some embodiments of the expression vector of paragraph 1, the one or more regulatory sequences includes a promoter, and the insulator sequence is placed upstream of the promoter.
3. In some embodiments of the expression vector of paragraph 1, the one or more regulatory sequences includes a terminator, and the insulator sequence is placed downstream of the terminator.
4. In some embodiments of expression vector of paragraph 1, the one or more regulatory sequences include a promoter and a terminator, and a first insulator sequence is placed upstream of the promoter, and a second insulator sequence is placed downstream of the terminator.
5. In some embodiments of the expression vector of any of paragraphs 1-4, the insulator sequence is a portion of an rRNA gene.
6. In some embodiments of the expression vector of any of paragraphs 1-5, the insulator sequence has a predicted histone occupancy probability of less than 0.3.
7. In some embodiments of the expression vector of any of paragraphs 1-5, the insulator sequence has a predicted histone occupancy probability of less than 0.2.
8. In some aspects of the expression vector of any of paragraphs 1-7, the fungal cells are filamentous fungal cells.
9. In another aspect, an integration cassette for expressing a gene of interest in fungal cells is provided, the integration cassette being comprised of a contiguous DNA sequence comprising:
   a site of insertion for a gene of interest desired to be expressed; and
   an insulator sequence upstream and/or downstream of the site of insertion, wherein the insulator sequence reduces histone binding;
   wherein upon introduction of the integration cassette to fungal cells and upon introduction of the gene of interest at the site of insertion, the presence of the insulator sequence increases the expression of the gene of interest compared to the level of expression obtained using an equivalent expression cassette lacking the insulator sequences.
10. In some embodiments of the integration cassette of paragraph 9, the integration cassette further comprises a promoter, and the insulator sequence is placed upstream of the promoter.
11. In some embodiments of the integration cassette of paragraph 9, the integration cassette further comprises a terminator, and the insulator sequence is placed downstream of the terminator.
12. In some embodiments of the integration cassette of paragraph 9, the integration cassette further comprises a promoter and a terminator, and a first insulator sequence is placed upstream of the promoter, and a second insulator sequence is placed downstream of the terminator.
13. In some embodiments of the integration cassette of any of paragraphs 9-12, the insulator sequence is a portion of an rRNA gene.
14. In some embodiments of the integration cassette of any of paragraphs 9-13, the insulator sequence has a predicted histone occupancy probability of less than 0.3.
15. In some embodiments of the integration cassette of any of paragraphs 9-13, the insulator sequence has a predicted histone occupancy probability of less than 0.2.
16. In some aspects of the integration cassette of any of paragraphs 9-15, the fungal cells are filamentous fungal cells.
17. In another aspect, a method for expressing a gene of interest in fungal cells is provided, the method comprising, introducing to the fungal cells
   (i) a contiguous DNA sequence comprising a region encoding a gene of interest desired to be expressed and one or more regulatory sequences sufficient to cause the expression of the gene of interest in the fungal cells; and
   (ii) an insulator sequence upstream or downstream of the contiguous DNA sequence in (i), wherein the insulator sequence reduces histone binding;
   wherein the presence of the insulator sequence increases the expression of the gene of interest in the fungal cells compared to the level of expression obtained in the absence of the insulator sequences.
18. In some aspects of the method of paragraph 17, the contiguous DNA sequence and insulator sequence are introduced simultaneously into the fungal cells.
19. In some aspects of the method of paragraph 17, the contiguous DNA sequence and insulator sequence are introduced sequentially into the fungal cells.
20. In some aspects of the method of any of paragraphs 17-19, the gene of interest and regulatory sequences are introduced simultaneously into the fungal cells as a contiguous DNA sequence.
21. In some embodiments of the method of any of paragraphs 17-20, the gene of interest and regulatory sequences are in an expression cassette.
22. In some aspects of the method of any of paragraphs 17-19, the gene of interest and regulatory sequences are introduced sequentially into the fungal cells to form a contiguous DNA sequence.
23. In some embodiments of the method of any of paragraphs 17-22, the regulatory sequences comprises a promoter, and the insulator sequence is placed upstream of the promoter.
24. In some embodiments of the method of any of paragraphs 17-22, the regulatory sequences comprises a terminator, and the insulator sequence is placed downstream of the terminator.
25. In some embodiments of the method of any of paragraphs 17-22, the regulatory sequences comprise a promoter and a terminator, and a first insulator sequence is placed upstream of the promoter, and a second insulator sequence is placed downstream of the terminator.
26. In some embodiments of the method of any of paragraphs 17-25, the insulator sequence is a portion of an rRNA gene.
27. In some embodiments of the method of any of paragraphs 17-26, the insulator sequence has a predicted histone occupancy probability of less than 0.3.
28. In some embodiments of the method of any of paragraphs 17-27, the insulator sequence has a predicted histone occupancy probability of less than 0.2.
29. In some aspects of the method of any of paragraphs 17-28, the fungal cells are filamentous fungal cells.
30. In another aspect, a gene of interest expressed by the method of any of paragraphs 17-29 is provided.

These and other aspects and embodiments of the present compositions and methods will be apparent to the skilled person in view of the present description. The following examples are intended to further illustrate, but not limit, the strains and methods.

### EXAMPLES

### A. Identification of a putative filamentous fungus insulator sequence

The non-coding regions (excluding promoters) of corresponding, presumed highly-expressed rRNA genes from different species of filamentous fungi, *i.e*., T. reesei, T. virens, and T. atroviride, as found in the U.S. Department of Energy Joint Genome Institute (JGI) website, as well as different *Aspergilli;* as found in the Broad Institute website and the CADRE website, were manually (visually) inspected for low degrees of sequence conservation using a web-based sequence analyzer. Genomic regions were selected that contained genes known to be highly expressed, such as the rDNA locus having the sequence of SEQ ID NO: 1. DNA stretches with high AT content, and flanking the rDNA locus, were selected for further study. Portions of this sequence (*i.e*., SEQ ID NO: 2 and SEQ ID NO: 3) were analyzed using the histone occupancy predicting software available online from the Segal Laboratory of Computational Biology (Weizmann Institute, Rehovot, 76100, Israel).

Subsequently, a visual examination was performed on the selected sequences. Attention was given to sequence conservation, or lack thereof, and nucleotide composition. A particular ~300-bp region (rDNA7; scaffold 9 of the U.S. Department of Energy Joint Genome Institute (JGI) website database; SEQ ID NO: 4) having 98% AT-content was selected as a candidate sequence. For this sequence of DNA, the predicted probability of occupancy using the Segal method was zero (Figure 1B).

The rDNA7 insulator DNA sequence was synthesized by GeneArt (Life Technologies, Carlsbad, CA, USA) and provided in a plasmid. Primers rDNA7-FW (SEQ ID NO: 5) and rDNA7-REV (SEQ ID NO: 6) were used to amplify the insulator sequences, which were duplicated by ligation to produce a 612 bp fragment, and cloned either (i) upstream (*i.e.,* 5'-insertion) of the cbh1 promoter (Pcbh1), using the *Msc*I restriction endonuclease site, (ii) downstream (*i.e.,* 3- insertion) of the cbh1 transcriptional terminator, using the *Bst*XI restriction endonuclease site, or (iii) both upstream of the cbh1 promoter and downstream of the cbh1 transcriptional terminator (*i.e*., 5' and 3' insertions), all in plasmid pTrex3gM-AkAA (similar to the pTrex3gM plasmid described in U.S. Patent Publication 20110136197 but with a different gene of interest) using standard molecular biology methods techniques. AkAA α-amylase (*i.e*., *Aspergillus kawachi* acid-stable α-amylase; US Patent No. 7,332,319) served as an exemplary protein of interest and reporter protein. All plasmids were sequenced to verify the presence of the 612-bp insulator sequence.

The sequences described, above, are shown, below:
SEQ ID NO: 1 - rDNA locus and flanking regions of *T. reesei* 
CAAGGTTGGATTCGATGATTCAATGGCGCACATGCCTCAGCCAGTGGCGAAGGAGGGAGAAGCT
GATTGGTCATCACCCAAGGGTTCCACTACACAGGCAGACGCGGTGTCACGTGACTATGCAAGCA
CTTTACGTGTGTTCAGAGCAGGTACGTCATCAAGGAAGGTGCCGAGATCTTTCTGGTGATGCCA
CGTCAATAATCTCAATCTCAACTGCAGAAGTAGCTTTATGGTGACTGAAGGCTCCATAAGATAT
TCGTGCTTTACGTCGCGATCTTAATTCGGGGCATATCCAGGTGAAATTTTGAAGCTTACATGCA
CTCTGCGGGACTGCATTGAAAGAGCATTCAATCTACTAGAAACCAAGTCAGGGTGCTCGATACC
TCGGCACTTCCTCTTTACCTACCTAGATTCACAAGGGCGACAGTCTCTCCTCGTATTGAACCAG
TTCGCCCAGGAGATGCTGTCGATACCCAAAGCGCCTTTGGTACCTGCGGCTCTTGCATTATGCA
TCTATCCCCTAACGCAGTTTCGCTTATCCGAGTTCCGGACCTATTGTAAAAGTGCCTACCCTG
GCGTTGCTTCAATTCGAGCTCGCGATATTCATTCATGAAATCCATGATTAGTGAGATTGATTGC
CTCGGACCAAGATCAAATCATCAGCTGGCCACATATCATGTTCTTACCAACGGCCTGATCCATA
CCCACAGACACACAGTTGTGCGCTGTAGCGCCATATTCCGAAACTGCCCCTCACACATCGCATC
TTTGAATACCACCACACGATTCTCCCAACTTCCTCCTCCCAGCCAGTCACAGTGACCCTCTCCG
TGAGTGAGGCTTCGATATGTCAAGCTGGACTAGGCTATTTGGTCGCCGCATTCCATGCTCATGC
TTCGCCCGCCCTTATACACCAGATGTTCGCAGACTCACCAAACCCATGGCGGCTTATTCTACAC
AATCTCGCCAGCCCGCATGGCTCACATCGCTCTTGAGCAATACAAGCCCGGCCCCCAAATTGTA
CGCCTGGAGCCCAGACAATTTAAGGGGTAGCGTTCCAAGCAGTCTGAGTCCTAGTGCAGACCAG
CCTGATCCTTCTGACCTGGATCGCCGAATCTTCATATTGGGGATTGGCAATCTGGGTCGCCTCT
ACGCTAGCTGTCTCTCCATGCACTCGCCTCGGCCTCCAATTACCCTCGTCGTACACAAACAGGA
 
ACTATTGGCCCAGTGGATCCATGGCCCAGGCCTCGAGATCACCAGATCAGGACGGCTTTATCGC
GGAAAAGACGAGTTTCAGATCGAGTTGTGGACTGAGCAAGCACCTACACATGGCCCTGTCAGAG
AAGTCGCTCACGCGAACAAAATCCGCAACCTCATCGTTGCAACTAAAGCCTCCGCAGCCATGAC
TGAAGCTGACAGACTACGACGCTATCTCGACAGCAGCAGCAGCGTGACGTTTCTTCAAAACGGC
ATGTCTAAACTGTGGCCGCCCTACGGACAGAAATACGTGGCCCAGCGGTACCCTGATAACGACG
CGCCCAACTTTCTGGCTTGTGTTACGAATCACGGCGTCTTGTCGGAGGGGCCATTCAAGAGTCT
TCACGCCTCACCTGCAGACAGTGCCGTTGGGCCTGTGTTGCTGAACAACACGGCTCGTCCTCTC
GCACCGAGCGTAGCCTACTTGACAGACGCCATTGCCTCCGCTCCCTGTCTCAACTCAAGGTCCT
TGACAAGAGCCGAACTCTGGGCATCGCAGCTTCAGAAGCTCGTTGTGAACTCTACCATAAACCC
CCTTACTGCCGTCCTTCGCTGTAAGAATGGCGCCCTCTTCGAAAACTCCAATGGCATTGTAGCC
AAAGTCATTGTCCGCCTTCTACATGAATCAAGCGCCATCCTGCAGACTCTCATCAATCATCCCA
TCGCCACCGAGATTCTCGCGGCGTCGTCAGATGTCAAGCCTGGCAAGTCTCTGGAAGCCGTGAG
AGAAGAGCTCTCTTTGCGCTTTTCATTTGCTGAGCTTCACTCAATGCTTTACAACATCGGCCAA
GTGGTAAAGGACAACACTAGCTCAATGCTTCAGGATGTTCGCGCAGGCAAACCAACGGAGATCC
ACGACTTCAATGGCTGGCTCGTCGAGACGGCTGATTTTCTGGAGCAAAGTAGCATCGGTCAGCC
CCGTGAACTGGACGTGTCTTGTCATCGGACGTTAATTGCGATGGTTGAGTCGGGCACTATTGTG
GACGACACCGAACTGGCCAAACAACTGCTACCCGGAATGTCTCATACATGGTAGAAAGACAAAA
CATTCATGCATGTGAAGCGAGAACATGGGGTATATATACTACATATCGATGACAGAAACACCAA
GCAATAGTAGCAGAACGTGGCAATGGTTTACATAGACAGAACTGTTTGGATACTGGATAGCTAT
TCCGCCAACATGCCTTATCCGTGATTGATTAACATACAACACTCCCCGATATAATCCCAGGATG
CTACTGATCATTTCATATATATATTGCTGTTCTCACTGTCATCTATCGCTCCGCGATATTGATA
TGCCACATTCAGCTCTCATGTGAAAAGGAAAATTGCGAGGCATCATCAAGATCATCGTTGAATA
GCTCCCACACGCGATTGCAGCATCCCATTGGTGTGACACCATTCCCAACTCGCTGCGCAACAAT
GCGCCATTTCTCGTCCTCGTAGCTCTTCAAAGCGTCCAAGAGCCTTTCGTCCTGCGATAATCAC
TGGGTCAACTTTACTCCTGTAGAGTGCAGGGTACATTCTTAGCGAAATCAGGAAAAGGAATTAC
ATACCATCTCTCTTGTCCACGATGTGCCCTCCGTCTTCAACCTCGTACAGTATCTCACCTGCAA
TGTGCCTGGATTACGTCCTGGGAAGAAGTCGGCAATTTGCTTCCACGTAAGATGCCTCTTTTCC
TTTAGCTCAATCAGAAGCTCGTCTTCCTCCTTGCTAAACTTGACCCTAGGTCGCTGAGGCCGCC
GCGCAGGAGAAGGCATCCCTTCTTGTCCAACAAGGTTATACGAATCTTGACCATGCTCTTCGAG
CATCGAAGCTGATGAGCGACCCGTATCTGGTAGATGATGGTGATGGTGGGACGGCCGAGGCATG
GATGACATGCTACTGTCCGGCACTCCATAGGGTCCGTATATGTAGGAAGTGGCAGCATCCGCGT
GTGCAGTCCTGGAGCCGACATAGGACGAGTTTTGCGAGTAGGGATCATCGGAATAGCGTTTTTG
ATCGTCTGTTACTGAGTAGTAAGGACTATAAGATGGTGCTGTCATCGGAGTGAGATTTGTCTCG
TGTAAATGCAACCTTTCGTTGTAGCTCATGTTTCTGAAATAAGAGGATGGGGTGTTCTGTAGCT
TCAGTTGCAATGATGCAGTTACAGCGTCTGGCTCAGAAAGCCACGTCGAGATAATGATCATGA
 
GATGTCCTCATTTATAAACCAAAAGTTCACGGCATTTTGCCCAAGTCAAAATCAGCCATATCCC
GTCTCTCCTGTTTCTCTCTTCCCCGCTCGGTTCTATCCCCTCTCCATGCGTCTTCCTTGTTCTC
CTCTGCTTCCTCGATGTCCTTTACCTATCGATACAAGGAAATTCGCCTCCGCATAGGCACATGC
CGTGAACTACAGCTTCATGCCGCGGTAGACAAAGAGTGCAAGTTCCGCGATATGGCAGGTATTG
AAGAGACATGGTGTTGAGCACGGCTCACCGGAGCAGAGACAGCCCGGATTAAGGCTGGAGATGA
GGTGCCCTCCATCACCCCGAAGTGGAAAGAGGATCTTGTGAAATTGTCCGATGTTGTAGATTCC
GTCACCGAATCGAGTGTCTGTGGAGAACCTCCCTTTGGACAACGGACGTTGTCCCATCTCCTGC
ACGCAACGGGTCCAAGCACGATTTTTCCAAGAGCAGTTACCGATGGGCAGGATTGAGAGCTCGG
ACGCTGGTTGCGGCAAGGAATGGATCTGTTGCTGTTGATGGGATGCATTGGCCGCGGTGGACAC
AATGAGTTTATATCCGGACTGCCTCAACCATGTAAGCCCGGCCTTGATACGAGACAAAATGTTA
CGATGTGACCATGGATACGACGCGCTAACGCAGCTCACGGACACTTCAAGCGCGGACCGACGCA
CCGTCAACAAGGTGGTAGGCGATCCTAAGGTGGCAATATGAAATACCAGAACAGGTAGGTAAAC
CCAAGATCGATAGCAATGCAACGGCGATTCTACTGCGGCTCACGATTATCTAGTACCTGCATTT
TAGGTTAAGGGGGTTTAACTAAAGGAACTATAGTAAGCCTTAGTTATAATAATATATAGCAGCA
GCCTTTATATTATTATTATAATTAAAGTATTATAAAAGCTAAGTTTATAAAAAATTAAGTATTA
TTTTATATAACTAAGTTAGTATATATTTAATAAAGTATATAATACTATTAATAAATAATTTAAA
AAATAAAAGAATTTTAATTTTACTTTAAAAAAATTATATTATTACTTAATTTAAATTATTTTAA
TTATTAAGTTTAAATATATATTTCTTATTTAAGTTATTTAAGTTATAACTAAGTATATAAATTA
AAAATAAATAAATTAAATTACTTAAGTTAAAATTAAATACTTAGTAAAGCTAAGGCTTTAGCTT
AATAATTATTTATTATTTAAGTTTATAAGTTAATTTAGCTAATATTATTATTATAAAAGTCTTA
GTTTTTTTTAAAAAAAATAAAGTTTTTATTAATATAAATAAATATTATTATAATTAAAATTT
ATTATAATTAAGCTTTATTATAAATAATTTTATATAAAAATAATAAATATTAAAGCTATTTATA
TAATATTATATTTATAAAATATAAAATAAATTTATTATAATTATTATAATTATTATTTAATTAT
TAATATATAATACTTTTATTATTTTATTATAATTTTTTACTTTTAATTATAAAATATATTTTT
TTATTATAATTTTAAACTTTTAAATAATTATTAAAAAGATTTTAAAATAAAACTAAGCTTTAAT
TCTTATAAAAAAAGTATTATTACTTAAGTAATATATTTTTACTATTTAAGTAATATACTTACTA
TAACTAGCTTAAATTAATATTAATTTATATAGCTTAATAAATTATAGTAAAATACTATATTATA
GCCTAGTAATTAAATAAGCCTTTTTTATAATAAGTATTACTTATAATAGTAATATAAAAATATA
TAAAGTATACCTAGGCTATTATTATTCTTAATAAAGCGCTTATAATAAAAGTACTAAAACTATA
ATAATAGTAATAATAATAATAATATAATTTTAAATTAAAATAAAAAGGTTATTAAAGGCATTTT
ATAAAATAATATAATACTTAGTATATTATATTATAATATAGTAAGTTAAGCTAGAATTTAATAG
CTTAAAGCTAATATAGCTAGCTATAGTAAGTATATTACTTAAATAATAAATATATACTACTTAA
GTAATAATACTTTTATAATATTAATACTTTTAATAATTATAAAACTATTTTTACTATTATATAT
TATTTTTTACTTATTAAGTATTATTATTATTTATTTATAAAATATTTTATTATAATTACTATTT
TTATAAAACTTATTATTATTAATTTATTATTTAATAAAACTAAATAATAATAAAATTACTAAAA
 
TATTTTATAAATATTAAATATTATTAAAGTAATTTAAAAAGTTTTAAAATAAATATTATTAATA
AATTTTACTTAATAAATTATAAAATTTAAATAATATAATATTTATAAAGTTAAAATAATAATAA
TAAAAATATTATATTATAACTTAAGCTTTTTTTTAATTTAATATATTTAATTAAGTAACTTAA
AGTATTAATTAATTATATATAAAGTTTATATAATATAATTTTATTTTTTTAATAAGTAAGTATA
GCTAGTAGTTATATAAGTTATTAATATATATATACATATATATATATATTAATAATTCTTTA
GTCCCTAGTCCCTAGGGCCCGCCCCGGGTCGCCCGCGGCCGCCGGCAGAGGCTGGGGCTGCTGG
GCAGGAGGAGGGGAGCAGAGGGGGCGGGCGGCATAGAGGGACGGGTGGCGACCCGAGGAGATGC
GTGCCCATTGCGAATCGTCGACCCGTGCGAGAAGAATGTCCAAAGGTTGGTCGAATGAATCTTC
CGTTCGGTTTCGTGCCTTTTCTTCTTCTTTTCTTTTTTGACTTCATCCATCTCCTGCCCGCCCG
CTTCCTCGACAAGCCCGCACCTACCCTACAGCCTAGCCCGTACCTACCCTATACCTACCCTGTA
CCTACCCTGGAGCTCCGTACAAAATGGCAGCCGGCCACCCGACGGCGTACCAAAAGCCCAAAGC
CCGAAGCCCGAAGCCCAAGACACGCAACCGGAGACCAGAGACCAGAAACCAAAGAAACCAAAAA
AAAAACCCAAGCCCAGACCCAAACGGCGACCTGGCCCAGCGCCTACCCTATACCTACCCTATAC
CTACCCTATACCTACCCTAGAGCCCTGCAAAACTCTCGGCCGCCTGCCTACCCTATACCTACCC
TATACCTACCCTAGAGCCCTGCAAAACTCACAGCCAGCTACCCTATACCTACCCTACACCTACC
CTAGAGCTACCCTACACCTTTCGGCAGAGTGCCTACCCTGTACCTACCCTGTACCTACCCTGTA
CCTACCCTGTACCTACCCTGGAGCCTGCCGGCGACTCTAGGGTAGCCGCCCCCCTCGGGACTTG
GCCGCTCCTTCCGCCCCCTCTCTCCCCCTCTCCCCCTCCCCGGCTCGCGCCCACGTCCCCCAAG
ATCTCCGCCAATCCTCCCCCCCGCCCCCCGCCTACCCTGGAGCTCCCCTTGGACCCTCGCCCGG
CCCCCTCATGGGACCTCCGAGCGCGGGCGCCCCGGCCTGCCAGGCTTGGGCTCGCGGCCGCG
TTGCGAATTTCCGCAAAAAGATTTTGTATGGGATTCCGCCCAAATCGACTCACGAAGAGACCGC
CTACCCTATACCTACCCTAGAGCAGGACGCTCGCGCGGGAGCGCCTACCCTAGAGCTGAGCCCG
AGCCGCGTCGCCGCGGCCCAGAGCCTCTCTCTGGGCGCCAACCACCAACCATCCCCTACGCGGC
CCCCCCGGGAGACTTGTCCCTTGGGAGGCCCGAAAACGTCGGGGAAATCGAGGCGAAGGACGGC
TTACCCTCAGCAGATCGTAACAACAAGGCTACTCTACTGCTTACAGGACCGTTTCGCACAGCTA
AGTCGTCTGCAAAGGATTTAACCCCGCTCGTGTTGAAATTACAATACGCGAAATACCGCGGCCC
GCTTCGAGGACCGCGGAGGAATCGCCTGCTGAGACACTAAGCCGAAGCCTATTCTTGTCCGTCT
ATACGTGCGGGTGCTATCACCGCGTTCTGGCATGGATTCTGACTTAGAGGCGTTCAGCCATTAT
CCAGCAGATGGTAGCTTCGCGGCACTGCCCGGTCGGACAGCCGCAAAAACCAATTATCTGAATG
AGCGGTTCCTCTCGTACTAAGCTCAATTACCATTGCGGTGAGGTCATCAGTAGGGTAAAACTAA
CCTGTCTCACGACGGTCTAAACCCAGCTCACGTTCCCTATTAGTGGGTGAACAATCCAACGCTT
ACCGAATTCTGCTTCGGTATGATAGGAAGAGCCGACATCGAAGGATCAAAAAGCGACGTCGCTA
TGAACGCTTGGCCGCCACAAGCCAGTTATCCCTGTGGTAACTTTTCTGGCACCTCTAGCCTCAA
ATGTCGAGGGACTAAAGGATCGATAGGCCACACTTTCATGGTTTGTATTCACACTGAAAATCAA
AATCAAGGGGACTTTTACCCTTTTGTTCTACTGGAGATTTCTGTTCTCCATGAGCCCCCCTTAG
 
GACACCTGCGTTATGGTTTAACAGATGTGCCGCCCCAGCCAAACTCCCCACCTGACAATGTCTT
CAACCCGGGTCGGCCCGCGAAGGACCTTAATACCAGAAGTTGGACGTAAATCCAGCCCCGCTTC
ATTGAATAAGTAAAAAAACAATAGGAGTAGTGGTATTTCACCGGCGCCGAAGCTCCCACCTATT
CTACACCTCCTATGTCTTTTCACAATGTCAAACTAGAGTCAAGCTCAACAGGGTCTTCTTTCCC
CGCTGATTCTGCCAAGCCCGTTCCCTTGGCTGTGGTTTCGCTAGATAGTAGATAGGGACAGTGG
GAATCTCGTTAATCCATTCATGCGCGTCACTAATTAGATGACGAGGCATTTGGCTACCTTAAGA
GAGTCATAGTTACTCCCGCCGTTTACCCGCGCTTGGTTGAATTACTTCACTTTGACATTCAGAG
CACTGGGCAGAAATCACATTGCGTCAACACCACTTTCTGGCCATCGCAATGCTATGTTTTAATT
AGACAGTCAGATTCCCCTTGTCCGTACCAGTTCTAAGTTGGTTGTTAACCGCACGCCGGACGGC
CGAAGCCTGCAAGGACATCAGCACCCAGATGCTGGCAGCCGCCGGCCGGTTGCCCGGCTAGTG
GCCGCCTGCTCCCGAGGCGTCCGTCCAAAGCCCAACGTGCCCAACCCTTAGAGCCAATCCTTAT
CCCGAAGTTACGGATCTATTTTGCCGACTTCCCTTATCTACATTGTTCTATCAACCAGAGGCTG
TTCACCTTGGAGACCTGCTGCGGTTATGAGTACGACCTGGCGTGAGAATTATTCCTTCCCGCGG
ATTTTCAAGGGACGTCGAGGGCGCACCGGACCCAGCAGAGGTGCTGGGCTCTTCCAGCCATTAA
ACCCTAGCTCCGGACAAACCGATTTCAGGGTGATAGACTGTTAAGAAGAAAAGAGAACTCTGCC
CGGGGCCCCCGCCGTCGTCTCCGCGTTCAGTTGCGTTGCCGCGCAAAACCCACATCCAGGTGCC
GGAATATTGACCGGCTCCCCTTTCGCCAAACGGCGCGAAGTGCGCCTTTGAAACGGAACTTCCC
TATGGCTTAGGATCGACTAACCCACGTCCAACCGCTGTTCACATGGAACCTTTCCCCACTTCGG
TCCTTCAAGTTCTCATTGAAGTATTTGCTACTACCACCAAGATCTGCACTAGAGGCCGTTCGAC
CCGGGCTCACGCCCTAGGCTTCGTCACGACCTCCACGTCCGCCTACTCCTCGGGGCATCGTTTC
TACCCCGAGGGCGAGGTATGGGTGAGACGCTTGAGCGCCATCCATTTTCAGGGCTAGTACATTC
GGCAGGTGAGTTGTTACACAGTCCTTAGCGGATTCCGACTTCCATGGCCACCGTCCTGCTGTCA
AGATGTACTAACGCCTTTTGTGGTGTCTGATGAGCGTCTACTCTGGCACCTTAACCTCGCGTTC
GGTTCATCCCGCATCGCCAGTTCTGCTTACCAAAAATGGCCCACTAGTGTTGATACATTCGAAT
GCCCACGTTCAATTAAGCAACAAGGGCTTCTTACATATTTAAAGTTTGAGAATGGATGAAGGCA
ATATAGCGCCCCCGGGTCCCTAATCATTCGCTTTACCTCATAAAACTGAGTTCAACACTGCTAT
CCTGAGGGAAACTTCGGCGGAAACCAGCTACTAGAAGGTTCGATTAGTCTTTCGCCCCCATGCC
CATATTTGACGATCGATTTGCACGTCAGAACCGCTGCGAGCCTCCACCAGAGTTTCCTCTGGCT
TCACCCTATACAGGCATAGTTCACCTTCTTTCGGGTCCGGCCCCGTATGCTCTTACTCAAATCC
ATCCGAGAACATCAGGATCGGTCGATGATGCGCCGAAGCTCTCACCTGCGTTCACTTTCATTAC
GCGTAGGGGTTTGACACCCGAACACTCGCATACGAAGACGACTCCTTGGTCCGTGTTTCAAGAC
GGGTCGCTGGTGACCATTACGCCAGCATCCTTGCAGATGCGCGGTCCTCAGTCCACCGCAGGGT
ATTATGCAACGGGCTATAACACTCCCAGGGGAGCCACGTTCCCGAAGCCTTTTTCCCCCGCGAC
GAACTGATGCTGGCCTGGACACGGCGAAGTGCACCGGGGAGAACCCCGGATGATCCGCCGCGCC
CAAGTCTGGTCACAAGCGCTTCCCTTTCAACAATTTCACGTACTATTTAACCCTCTTTTCAAGG
 
TGCTTTTCATCTTTCGATCACTCTACTTGTGCGCTATCGGTCTCTGGCCAATATTTAGCTTTAG
AAGACATATACCTCCCATTTTGAGCAGCATTCCCAAACTACTCGACTCGTCGAAGGAGCTTTAC
AGAGGCTCGGCGGCCAGCCAGACGGGGCTCTCACCCTCTGTGGCGTCCCGTTCCAGGGAACTCG
GGCGGCGCCTTGCCAAAAGCATCCTCTACAAATTACAACTCGGACCCGAAAGGGCCAGATTTCA
AATTTGAGCTGTTGCCGCTTCACTCGCCGTTACTGGGGCAATCCCTGTTGGTTTCTTTTCCTCC
GCTTATTGATATGCTTAAGTTCAGCGGGTATTCCTACCTGATCCGAGGTCAACATTTCAGAGTT
TGGGGTGTTTTACGGCTGTGGCCGCGCCGCGCTCCCGGTGCGAGTGTGCAAACTACTGCGCAGG
AGAGGCTGCGGCGAGACCGCCACTGTATTTCGGGGGCGGCCCGGTGAGGGGCCGATCCCCAACG
CCGACCCCCCGGAGGGGTTCGAGGGTTGAAATGACGCTCGGACAGGCATGCCCGCCAGAATACT
GGCGGGCGCAATGTGCGTTCAAAGATTCGATGATTCACTGAATTCTGCAATTCACATTACTTAT
CGCATTTCGCTGCGTTCTTCATCGATGCCAGAACCAAGAGATCCGTTGTTGAAAGTTTTGATTC
ATTTTCGAGACGCCCGCTAGGGTCGCCGAGAAAGGCTCAGAGCAAAGGTAAAACAGAGCCGCGA
CGGAAGCCGCGACGGAGAGAAAAAAGAGTTTGAGTTGGTCCTCCGGCGGGCGCCATGGGATCCG
GGGCTGCGACGCGCCCGGGGCAGAGAATCCCGCCGAGGCAACAGATTGGTAACGTTCACATTGG
GGTTTGGGAGTTGTAAACTCGGTAATGATCCCTCCGCTGGTTCACCAACGGAGACCTTGTTACG
ACTTTTACTTCCTCTAAATGACCGAGTTTGGAGAGCTTTCCGGCCCTGAGTGGTAGTTGCCCAC
CTCTCTGGGCCAGTCCGGACGCCTCACTGAGCCATTCAATCGGTAGTAGCGACGGGCGGTGTGT
ACAAAGGGCAGGGACGTAATCAACGCAAGCTGATGACTTGCGCTTACTAGGGATTCCTCGTTGA
AGAGCAATAATTGCAATGCTCTATCCCCAGCACGACAGAGTTTAACAAGATTACCCAGGCCTTC
CGGCCAAGGGAGTACTCGCTGGCTCTGTCAGTGTAGCGCGTGCGGCCCAGAACATCTAAGGG
CATCACAGACCTGTTATTGCCTCAAACTTCCATCGGCTTGAGCCGATAGTCCCTCTAAGAAGCC
GGCGTACTGCCAAAGCAATACGGGCTATTTAGCAGGTTAAGGTCTCGTTCGTTATCGCAATTAA
GCAGACAAATCACTCCACCAACTAAGAACGGCCATGCACCACCACCCACAAAATCAAGAAAGAG
CTCTCAATCTGTCAATCCTCATTGTGTCTGGACCTGGTGAGTTTCCCCGTGTTGAGTCAAATTA
AGCCGCAGGCTCCACCCCTGGTGGTGCCCTTCCGTCAATTTCTTTAAGTTTCAGCCTTGCGACC
ATACTCCCCCTGGAGCCCAAGCACTTTGATTTCTCGTAAGGTGCCGAACGCGTCAAAAATGTAA
CATCGTCCGATCCCTAGTCGGCATAGTTTATGGTTAAGACTACGACGGTATCTGATCGTCTTCG
ATCCCCTAACTTTCGTTCCTGATTAATGAAAACATCCTTGGCAAATGCTTTCGCAGTAGTTAGT
CTTCAATAAATCCAAGAATTTCACCTCTGACAATTGAATACTGATGCCCCCGACTGTCCCTATT
AATCATTACGGCGGTCCTAGAAACCAACAAAATAGAACCACACGTCCTATTCTATTATTCCATG
CTAATGTATTCGAGCATAGGCCTGCCTTGAGCACTCTAATTTTTCAAAGTAAAAGTCCTGTTT
CCCCGCCACACCCAGTGAAGGGCATGGGGTTCCGCAGAGGGAAAGGCCCGGCCGGACCAGTGCA
CGCGGTGAGGCGGACCGGCCAGCCAGGCCCAAGGTTCAACTACGAGCTTTTTAACCACAACAAC
TTTAATATACGCTATTGGAGCTGGAATTACCGCGGCTGCTGGCACCAGACTTGCCCTCCAATTG
TTCCTCGTTAAGGGATTTAAATTGTACTCATTCCGATTACAAGACCCAAAAGAGCCCTGTATCA
 
GTATTTATTGTCACTACCTCCCCGTGTCGGATTGGGTAATTTGCGCGCCTGCTGCCTTCCTTG
GATGTAGTAGCCGTTTCTCAGGCTCCTTCTCCGGGGTCGAGCCCTAACCCTCCGTTACCCGTTG
CCACCATGTTTGGCCAATACCCAAACATCGAAAGTTGATAGGGAAGAAATTTGAATGAGCCATC
GCCGGCACAAGGCCTGTCGATTCGACTAGTTATCATGATTCACCAGAGAGCCCCGAGGGGCATT
GGTTTTTAATCTAATAAATACATCCCTTCCGAAGTCGGATTTTCAGCATGTATTAGCTCTAGA
ATTACCACGGTTATCCAAGTAGTAAAGTATTATCAAATAAACGATAACTTATATAATGAGCCAT
TCGCAGTTTCGCGGTATAATTGCTTATACTTAGACATGCATGGCTTAATCTTTGAGACAAGCAT
ATGACTACTGGCAGAATCAACCAGGTAACTATCGTCAGGGCGTCCGGCACAGGGCCCTCGCGC
CCACCGGCGGAGTATCTCATCCGCCGGAGCGATTTCTAGCACACTAGGTCGGCCGCCGCGACGC
ACTGAAGCGTCAGCGCCGGCGCCCCCGCAACGCCGCCCAGGGCCACTGTTACATCGCCGGGA
CAGCGCTACGAGCGGCAATCCGCTCCCCCCGCCAAAGTTCCCAGGGCCACCCTCCTCGGCGCCA
AAGGCGCTGAATCGGGGGCCCAGAAGAGGGGCCCGTTCCCCAGCGTCTACCATTGCGGTGTCA
GCTGGGGGCGGGCTGCCCCCTGTTGCTCAGTGAAGGCCGCGTGCCACAGAGGGCCAGCAGTCCA
CCGAACCCAACCGACGGCGCCAGAGGCCCCTGAGGGGCGCCCCGACGCAGCCGTGCTTCCTCCG
TACGGCGTGCACCCGTGGGAGCCGCTTTCGGGAGGGGCGACAGATAGTTACGACCAATCGCGAG
CGAGAATTACATCCTGCAAGCCTGTTCAAGAGGTCGCCATAACTAAACGTCTACAAGATATGGT
ACAGTTTGATCGGTCGGGGGTTCGCCGGTCGTCACCTTTCGGCTGCCTTCCTTTGGTTGCCCCC
TTCCTCTCATAAGTCCTGCGAGCTAATGGAAGCCCGGGCCCGGGCAGGTTGGGCTCGTGGCCGC
GTTCCGATTTTCCGCAAAATTCATTTGTATGCGTTTTTGCCCAAATCGACTCACGGCCATACAA
ACGGGAAACCGACTTGGCCAGTTTTTTCGGACTTGGCCAAATTCGAGCCCTCCCGCTAGCCTGG
AGCTACCCTAGAGCCCCGCGCAAAGTGTGGGCGCCTACCCTGGAGCGCTATTTAGTCGCCCACC
CGGCGGCCGCTCCTGGGCCTGGGCCACGGCCGGGGTGGCCTACCCTATAGCCGCCGCCGGACCT
ACCCTGGAGCCCGCCCCGGGTCGCCCGCGGCCGCCGGCAGAGGCTGGGGCTGCTGGGCAGGAGG
AGGGGAGCAGAGGGGGCGGGCGGCATAGAGGGACGGGTGGCGACCCGAGGAGATGCGTGCCCAT
TGCGAATCGTCGACCCGTGCGAGAAGAATGTCCAAAGGTTGGTCGAATGAATCTTCCGTTCGGT
TTCGTGCCTTTTCTTCTTCTTTTCTTTTTTGACTTCATCCATCTCCTGCCCGCCCGCTTCCTCG
ACAAGCCCGCACCTACCCTACAGCCTAGCCCGTACCTACCCTATACCTACCCTGTACCTACCCT
GGAGCTCCGTACAAAATGGCAGCCGGCCACCCGACGGCGTACCAAAAGCCCAAAGCCCGAAGCC
CGAAGCCCAAGACACGCAACCGGAGACCAGAGACCAGAAACCAAAGAAACCAAAAAAAAAACCC
AAGCCCAGACCCAAACGGCGACCTGGCCCAGCGCCTACCCTATACCTACCCTATACCTACCCTA
TACCTACCCTAGAGCCCTGCAAAACTCTCGGCCGCCTGCCTACCCTATACCTACCCTATACCTA
CCCTAGAGCCCTGCAAAACTCACAGCCAGCTACCCTATACCTACCCTACACCTACCCTAGAGCT
ACCCTACACCTTTCGGCAGAGTGCCTACCCTGTACCTACCCTGTACCTACCCTGTACCTACCCT
GTACCTACCCTGGAGCCTGCCGGCGACTCTAGGGTAGCCGCCCCCCTCGGGACTTGGCCGCTCC
TTCCGCCCCCTCTCTCCCCCTCTCCCCCTCCCCGGCTCGCGCCCACGTCCCCCAAGATCTCCGC
 
CAATCCTCCCCCCCGCCCCCCGCCTACCCTGGAGCTCCCCTTGGACCCTCGCCCGGCCCCCTCA
TGGGACCTCTAAGCGCGCGGGCGCCCCGGCCTGCCAGGCTTAGGCTTTTAATAATATTATAATT
TTAAATATTTAAGTAATTAATTATTTAGAATTTTACTTAAATATACTTTAACTATTTATTATTT
TATTTTATTTTAAGTTCTTAATTTATTTATATTAAGTTTTATTTAATAATAAACTTAAAGTTTA
TTTTTAAAAAAATAATACTTTAATATTATATAATAATTTAATTATAAATAATATAACTTTATTA
AATTATAATTTCTTTTATTTATTAATTAAAGTTATTAATTATATATATATATATATATATATTA
AAAGTTTATTTTTTATTTTAAATAATAAAGTAAGTTATAATTAATAATATAATAATTTTTAATT
TTTAAAATTATTAATATATTTTATATTATAAATTTATAAAAAATATTTAAATTTATATTATTAT
ATAATTAAAATAAATTAATAATTTAATTATTATTTTTTTTAATTTTTAATTTTTTATATAAATT
TTAATTTTATATATTATTAAAAGTTATATATTTTTAATTTATAAATAATTTATTTAATAACTTT
TTAATAGCTAATTTAAATTATTAAATAAAATATATTTTTAATTTATAATTTTTATAACTTTTAA
TTATAATTAAATTTAAAAAAAATTATTAAATATAACTTAAGTAATATAAATAATTTAAAAGTTT
TTTAAGAAAACTTTTTAATAATTTTTTTTTAAATTTTTTTTAAATTTTTTAATATTTTTTTAAT
AATTTTTTAATAATTTCTTTTTATAAATTTAAAAGTTTTTTTAATTTTTATTTATAAAACTTA
TATTTTTAATAAATAAATATTATATTAAAATTTAATAATATTAATATTAAAAGTTATATAAAAT
TTTAAAATTATAAAGTATTATTATTATTATTATTATTATTTATTTACTTATTTATTTACTTTTT
TATTTTTAAATATATTTAATTTTAATAAATTATAAAAGAATTAATTTTTTTATATTATTATATA
TAATAAATAAAAAAATAAAATAAATTTATATTTAATAATTTATTAAACTTTTATAATATAATAT
TACTTAATAATATTATTATAAAAAAATAAGCTTATTATTAACTTAATTAAAAAATATTAAATAA
TAAACTTTATATAATATATATATTATATTATATATAATAATTATTATTTAAATAAAAATATATT
ATAATATAAAAAAGTATTAATATAAAAATAATAAAAACTTTATTAAAAATCCTTTTATTTATA
TTATAGTATATTTTTATTTAAACTATAGTAGTTTATATATAAAATATATAGTAGTTTATATATA
AGGCAAAATTACTATATATATACTATTAAGTCTACTACTATATACTTAATTATACTATAAGCTA
ATAGCAGGCTTGTTTTATACTATATAAGGTTATAGCTCTAGTATTCTTAATTATTACAGTTAA
CCTTAGAGCTAACCTTATTACAGTCAGCGACTTAATTATAGCGCGAGATTAAGAGCAGCACTTA
TTATTATTAGGATAAAGGAGGCAGCTGCAGCAGCAGAAGAGGCAGCAGCAGAGACAGCAGATAC
CCTAGTGTTAATCCTATATGCCCTCTATCTTATAAGTATAATATTCTTAACAGTAATTACTTCT
AGTATAATACTATATTTTAATACTTAAGTATAGGATATTTTACTTAGGAGGTTAGTCTAGCTAT
TAGTATAAACTACTGTAGTTTAAATAGAATTTTATAATAGTATAAATAGTAGGACTCTTCTCTA
GTAATAATAATATTACTTAATATTATTTTATATTATTTATTTAATTTTACTATCTTATATAGTC
TATTTATTAATGCCAGTCTTCTGTACCTATAGCACTAGCTATCCACCCTAGCTCACTGACAAAC
TCGTCAAGGCCTGCCTCAATTGGTGTATCCAACATGTTGGATCTGCTGGGAATGACCTTTGGTG
AGGCACGATGTGCAAAAGTCTGCGGAAGAAACGCGTCCTTGCTATCCTCGCCGTGTCGTGGCAA
GCTGTATCCCTGGAACGAAGACAGGGGCGACTGGGATAGAGGGGCGGAGGGAAGTGTCCCTCG
TCCTCGTTGCTGGACAAAATGTCGTCGTCATCATCTTCATCATCATAGAAGCTCATGGGGACAT
 
CTTCTTGAGATGGACTTCCGGTGATATGGTCTGATCTTGTGATAGGCCATTCATACTGCGGCCT
TGTTTCGTTCATGACGGCTGAGATAGGCTGGCTCTGCTTTGGTAATAGAGACGGTACTGTGAGG
CTGGGTTTGACTACTGTCGTTGAGGATCCGGCCTCGGTATTGTGTTTACAGTATCGGATGATG
AAGAGCGTTGAAAGGGGGGTGGCGAGAGAACAGTGGCAAACGGTAGACTTTCTGAGACCGCAGA
GGCTGCAAAATCCTCATCGTCCTCATCATGCTCGATGACATCCTCTGGTATCTTGAGCGGTGGT
AATTGAGCAGTGTGACTTGGGAAACGCAGGGGTTTGTGGCTCCCTTCCCGTAGGATTCGGGAGA
GTGAAGTTGGAGAAATGTCTCTTGGTCGGCACCCTCGCTGAGTGATGACATATGACGGCCAGA
GCGGGCATGTGGTGAGGGGACTGTGATAACGTTGTCGTAGGCGGTAAATATGGTTTCTTGCGAG
CAAGCAGAGGCAGCACGTTGACTGTCTGGTGTCTCCAGTTTGCGAGAAAAGAATCTCTTCCATG
AGCGTGGTGACGTTGAGCGAGACGATGAAGATGACGAAGGTTGTGGAGCTGACTGGGAACGTCG
TTTGTTTGTGTCCGGCGAGAGGGGCGTTGGTGGTAGCGGCTTTATAAATTTGGTTGCCGGGTCC
ATTGTCCTGAAATTTTCCAAGTTCACAGATGTCGTGGACGCACTTCTGAAGCGCGCCGTGCGTT
CGATGGGGACTTGAAGAGTATTGACTGTCTGACCGGGCATATATGGGCAGGCAGTTGTGTGAGG
CATCGTAGTATCAAATGTTTCGGTTGATCCGTCTACCTCGTACTATGGCGGCGCAAGTTAGATT
TGGAAAAAAAAAAAAAAATAGAAAAAAAAATAAAAAAAAATCTGATACTCTGTGTGAGACGGGT
GAGAATTGTTGGCTGACAAGGGGCAGAGAGTTGTTATACCCATGGATCAGGATCAGGAATTAAT
AACCTGCCCCAACGGCTGGGATACTCGACGAAGCGCATTGGGATGATAGCAGAAAGTGAAATTT
GTGACTTACATAGTAGAAGTACGTGTGACCCATCTTTAGGCCACCACTTGTCTTGGGATGGTGC
GGGCTCCCATCATCGTCCATGACGTTCTGGAATGCGAAGCATCCTCTCCATTGGCCCTGGCCAC
GTCTACTGTCCCGTTCCATGCTATAGCACATGGTGAAATTGTCCCAAGAGCCAATGAGGCGGAC
GGATCGGACAGATGGCTCCGTCTGACTGTGAGGTTGGGGCGTCAGCTTAGGTCTGTTCCAGCGG
GCTAGTGATGGGCTGATCTGAAGATTTGTAATTTCGAACTGAGGGTATCGGCAGGTGCAGAATG
CACGAGTGCGTCCGGGATTGGGAGAGGGGTAAGCCAGGAGTGAAGATTCAACTCGAAGAGATTC
CCGTCTATCTTGTGTGGCCCGCGGAATCCCACCGGGCAAGCATAAGACTGGCAACGAGAGCAGG
GTGTAGACATGAGGTGCAGTGTTGAAATGGCCACGGCGTAACTCATGAGTCCGTGAAGCCAATG
TGTAGGGCAAACCGGCATGAAGCTGCGCGGCAATCAAAGTTCAATGGACTCATACCAACGAGCG
AGCAGGTCAATGCAAGGGGTAGGCAATGTGAGCGGGCTGACGGCCACACTAATGAGCACTTGAC
CTGGCAAAAGCCAACAAAAAGGGAGTACTCCGTCCGGGCTTCTGAGAAAGCAGGGCCTAGACC
GGACAGAGTGAACAGCCGACCGTTCGAGGTGCGGGATTCAGTGAGACACGGAACACGCTGAGCC
AAAGGCCGAGTAGACCAAGGGCTACCAATGCGCCCTGAACGAGCTGAGCCAGAAAGCGAGAGAC
GATAAGTGGGAGGAACCGGTCGAGCCCGGTAGTGAGGAAGGGGAACGCTCGGGAGACAAAAGTA
AAAAGAAAGAAAAAGAGATAAAATAAAACACAGACAGATGTTGAGGATATTGAAGAAGGGAAAA
TGAAGGGAAAATGAAATCCAACCAAAAGACAAGGACAAAGGGGAAAAGAAGAAGAGTCCGCAAG
ACACTCACAACATAAATGTAACAAGAGTCGTTGTTGGGTCCATTGTGCTGAAAGGCGCGGGAGA
CAATCGACTCGAGTATCTGGTTTGGGTATAAGAGTCTTATGAGGGCCGATAGTAAGCAACCGAG
 
GACTGTCTCGTAATCCGGCGATTATCTTACAATGTCTCGCGCTAACGTTGGCTGGCAGAGAAAG
TCAACGTGCGAGTCTATTCTGATGCAGTATTCCTTGGGCGTCAGAACGGAGCAGAGTTGAGAGT
GAGAGTGAGCTTGAGTGCGTGAAACACGGCGGGCTGAATGGCATGTTTGAAGAAGCGCTGAAGC
GAATAGCGTTGTGAGGTGATTGGCTCAAGGGCGCGGTTTAACTCCATGTGCTGCAAGCGCCGCC
ATCTAGCCGTACACTGGCAAGTTGTACTAGATATGGTGGTGGAAACCGGTCCCTGGACGGTTGG
ATGAATTTTGGTCGTATTGCAGCGTTGGATGGAAGGGAGGAATAGGGGGGCGAGACCAAGAAG
GGAGAGACGGGCGCTTAGGACGATGAAAGAGAACGTCGTTAGACGCGATCCCGAAAGTCGACGG
GGGTTGTTCAAGTAATAAGTGGCAAAAGATTTTCACCAGAACGGATAGAAGCCGCGATCAGCAG
GGATTCTGAGCAATGAATCGGACGAGCTGAATTTGGCAAGAAGTGTTCCAAAGTGGCGATGAAA
AGATGGTATGAGTCTGGAGCTTCATGAGAGCGGTAGATTGTCAGACCAGAGGCTCAGTACAACA
GCTGCGATGCTGCGGGGTGGGAATGCAGCAGAAAGAGCGTGATGCTTTCCAGGCTGTTGATGCT
GATGTTGGTGACGACGGCAGATCTCTTGCGGCCCAGAGCTCAGTAATAGTTTCCCGGCCAGCA
GCGTGACGACAGAGGCTCCAAGGGTCAGCCGCCATAACACCCAGAGGTGGAATCCGGAGAGGCA
GGCTGACTAGCGAGCTGGCACTTGGAGGTGGTGTGAGCGATAGTGTTGTACGAACCAGCGATAG
TTTGGTTTTGGCGTGATGCAGACTCCGGGATGTTGTCTGGATTTCCGCATCTGAGTAACGGCC
TGGTCAACGGGACGGCTCCTCTGAGGTTGTGACAAGAGCAGACCTTGGTCGGAAAGCCGGCGC
TGTGCC
SEQ ID NO: 2 - Part of the 5' flanking region of rDNA locus of *T. reesei* 
GCCATATCCCGTCTCTCCTGTTTCTCTCTTCCCCGCTCGGTTCTATCCCCTCTCCATGCGTCTT
CCTTGTTCTCCTCTGCTTCCTCGATGTCCTTTACCTATCGATACAAGGAAATTCGCCTCCGCAT
AGGCACATGCCGTGAACTACAGCTTCATGCCGCGGTAGACAAAGAGTGCAAGTTCCGCGATATG
GCAGGTATTGAAGAGACATGGTGTTGAGCACGGCTCACCGGAGCAGAGACAGCCCGGATTAAGG
CTGGAGATGAGGTGCCCTCCATCACCCCGAAGTGGAAAGAGGATCTTGTGAAATTGTCCGATGT
TGTAGATTCCGTCACCGAATCGAGTGTCTGTGGAGAACCTCCCTTTGGACAACGGACGTTGTCC
CATCTCCTGCACGCAACGGGTCCAAGCACGATTTTTCCAAGAGCAGTTACCGATGGGCAGGATT
GAGAGCTCGGACGCTGGTTGCGGCAAGGAATGGATCTGTTGCTGTTGATGGGATGCATTGGCCG
CGGTGGACACAATGAGTTTATATCCGGACTGCCTCAACCATGTAAGCCCGGCCTTGATACGAGA
CAAAATGTTACGATGTGACCATGGATACGACGCGCTAACGCAGCTCACGGACACTTCAAGCGCG
GACCGACGCACCGTCAACAAGGTGGTAGGCGATCCTAAGGTGGCAATATGAAATACCAGAACAG
GTAGGTAAACCCAAGATCGATAGCAATGCAACGGCGATTCTACTGCGGCTCACGATTATCTAGT
ACCTGCATTTTAGGTTAAGGGGGTTTAACTAAAGGAACTATAGTAAGCCTTAGTTATAATAATA
TATAGCAGCAGCCTTTATATTATTATTATAATTAAAGTATTATAAAAGCTAAGTTTATAAAAAA
TTAAGTATTATTTTATATAACTAAGTTAGTATATATTTAATAAAGTATATAATACTATTAATAA
ATAATTTAAAAAATAAAAGAATTTTAATTTTACTTTAAAAAATTATATTATTACTTAATTTAA
 
ATTATTTTAATTATTAAGTTTAAATATATATTTCTTATTTAAGTTATTTAAGTTATAACTAAGT
ATATAAATTAAAAATAAATAAATTAAATTACTTAAGTTAAAATTAAATACTTAGTAAAGCTAAG
GCTTTAGCTTAATAATTATTTATTATTTAAGTTTATAAGTTAATTTAGCTAATATTATTATTAT
AAAAGTCTTAGTTTTTTTTAAAAAAAATAAAGTTTTTATTAATATAAATAAATATTATTATA
ATTAAAATTTATTATAATTAAGCTTTATTATAAATAATTTTATATAAAAATAATAAATATTAAA
GCTATTTATATAATATTATATTTATAAAATATAAAATAAATTTATTATAATTATTTATAATTATT
ATTTAATTATTAATATATAATACTTTTATTATTTATTATAATTTTTTACTTTTTAATTATAAA
ATATATTTTTTTATTATAATTTTAAACTTTTAAATAATTATTAAAAAGATTTTAAAATAAAACT
AAGCTTTAATTCTTATAAAAAAAGTATTATTACTTAAGTAATATATTTTTACTATTTAAGTAAT
ATACTTACTATAACTAGCTTAAATTAATATTAATTTATATAGCTTAATAAATTATAGTAAAATA
CTATATTATAGCCTAGTAATTAAATAAGCCTTTTTTATAATAAGTATTACTTATAATAGTAATA
TAAAAATATATAAAGTATACCTAGGCTATTATTATTCTTAATAAAGCGCTTATAATAAAAGTAC
TAAAACTATAATAATAGTAATAATAATAATAATATAATTTTAAATTAAAATAAAAAGGTTATTA
AAGGCATTTTATAAAATAATATAATACTTAGTATATTATATTATAATATAGTAAGTTAAGCTAG
AATTTAATAGCTTAAAGCTAATATAGCTAGCTATAGTAAGTATATTACTTAAATAATAAATATA
TACTACTTAAGTAATAATACTTTTATAATATTAATACTTTTAATAATTATAAAACTATTTTTAC
TATTATATATTATTTTTTACTTATTAAGTATTATTATTATTTATTTATAAAATATTTTATTATA
ATTACTATTTTTATAAAACTTATTATTATTAATTTATTATTTAATAAAACTAAATAATAATAAA
ATTACTAAAATATTTTATAAATATTAAATATTATTAAAGTAATTTAAAAAGTTTTAAAATAAAT
ATTATTAATAAATTTTACTTAATAAATTATAAAATTTAAATAATATAATATTTATAAAGTTAAA
ATAATAATAATAAAAATATTATATTATAACTTAAGCTTTTTTTTTAATTTAATATATTTAATTA
AGTAACTTAAAGTATTAATTAATTATATATAAAGTTTATATAATATAATTTTATTTTTTTAATA
AGTAAGTATAGCTAGTAGTTATATAAGTTATTAATATATATATACATATATATATATATATTAA
TAATTCTTTAGTCCCTAGTCCCTAGGGCCCGCCCCGGGTCGCCCGCGGCCGCCGGCAGAGGCTG
GGGCTGCTGGGCAGGAGGAGGGGAGCAGAGGGGGCGGGCGGCATAGAGGGACGGGTGGCGACCC
GAGGAGATGCGTGCCCATTGCGAATCGTCGACCCGTGCGAGAAGAATGTCCAAAGGTTGGTCGA
AT
SEQ ID NO: 3 - Part of the 5' flanking region of rDNA locus of *T. reesei* 
AGAGCTACCCTACACCTTTCGGCAGAGTGCCTACCCTGTACCTACCCTGTACCTACCCTGTACC
TACCCTGTACCTACCCTGGAGCCTGCCGGCGACTCTAGGGTAGCCGCCCCCCTCGGGACTTGGC
CGCTCCTTCCGCCCCCTCTCTCCCCCTCTCCCCCTCCCCGGCTCGCGCCCACGTCCCCCAAGAT
CTCCGCCAATCCTCCCCCCCGCCCCCCGCCTACCCTGGAGCTCCCCTTGGACCCTCGCCCGGCC
CCCTCATGGGACCTCTAAGCGCGCGGGCGCCCCGGCCTGCCAGGCTTAGGCTTTTAATAATATT
ATAATTTTAAATATTTAAGTAATTAATTATTTAGAATTTTACTTAAATATACTTTAACTATTTA
 
TTATTTTATTTTATTTTAAGTTCTTAATTTATTTATATTAAGTTTATTTAATAATAAACTTAA
AGTTTATTTTTAAAAAAATAATACTTTAATATTATATAATAATTTAATTATAAATAATATAACT
TTATTAAATTATAATTTCTTTTATTTATTAATTAAAGTTATTAATTATATATATATATATATAT
ATATTAAAAGTTTATTTTTTATTTTAAATAATAAAGTAAGTTATAATTAATAATATAATAATTT
TTAATTTTTAAAATTATTAATATATTTTATATTATAAATTTATAAAAAATATTTAAATTTATAT
TATTATATAATTAAAATAAATTAATAATTTAATTATTATTTTTTTTAATTTTTAATTTTTTATA
TAAATTTTAATTTTATATATTATTAAAAGTTATATATTTTTAATTTATAAATAATTTATTTAAT
AACTTTTTAATAGCTAATTTAAATTATTAAATAAAATATATTTTTAATTTATAATTTTTATAAC
TTTTAATTATAATTAAATTTAAAAAAAATTATTAAATATAACTTAAGTAATATAAATAATTTAA
AAGTTTTTTAAGAAAACTTTTTAATAATTTTTTTTTAAATTTTTTTTAAATTTTTTAATATTTT
TTTAATAATTTTTTAATAATTTCTTTTTATAAATTTAAAAGTTTTTTTTAATTTTTATTTATAA
AACTTATATTTTTAATAAATAAATATTATATTAAAATTTAATAATATTAATATTAAAAGTTATA
TAAAATTTTAAAATTATAAAGTATTATTATTATTATTATTATTATTTATTTACTTATTTATTTA
CTTTTTTATTTTTTAAATATATTTAATTTTAATAAATTATAAAAGAATTAATTTTTTTATATTAT
TATATATAATAAATAAAAAAATAAAATAAATTTATATTTAATAATTTATTAAACTTTTATAATA
TAATATTACTTAATAATATTATTATAAAAAAATAAGCTTATTATTAACTTAATTAAAAAATATT
AAATAATAAACTTTATATAATATATATATTATATTATATATAATAATTATTATTTAAATAAAAA
TATATTATAATATAAAAAAGTATTAATATAAAAATAATAAAAACTTTATTAAAAATCCTTTTA
TTTATATTATAGTATATTTTTATTTAAACTATAGTAGTTTATATATAAAATATATAGTAGTTTA
TATATAAGGCAAAATTACTATATATATACTATTAAGTCTACTACTATATACTTAATTATACTAT
AAGCTAATAGCAGGCTTGTTTTATACTATATAAGGTTATAGCTCTAGTATTCTTAATTATTAC
AGTTAACCTTAGAGCTAACCTTATTACAGTCAGCGACTTAATTATAGCGCGAGATTAAGAGCAG
CACTTATTATTATTAGGATAAAGGAGGCAGCTGCAGCAGCAGAAGAGGCAGCAGCAGAGACAGC
AGATACCCTAGTGTTAATCCTATATGCCCTCTATCTTATAAGTATAATATTCTTAACAGTAATT
ACTTCTAGTATAATACTATATTTTAATACTTAAGTATAGGATATTTTACTTAGGAGGTTAGTCT
AGCTATTAGTATAAACTACTGTAGTTTAAATAGAATTTTATAATAGTATAAATAGTAGGACTCT
TCTCTAGTAATAATAATATTACTTAATATTATTTTATATTATTTATTTAATTTTACTATCTTAT
ATAGTCTATTTATTAATGCCAGTCTTCTGTACCTATAGCACTAGCTATCCACCCTAGCTCACTG
ACAAACTCGTCAAGGCCTGCCT
SEQ ID NO: 4 - Portion of SEQ ID NO 3 selected for synthesis 
GAGCTCGAATTCATTAAAGTTATTAATTATATATATATATATATATATATTAAAAGTTTATTTT
TTATTTTAAATAATAAAGTAAGTTATAATTAATAATATAATAATTTTTAATTTTTAAAATTATT
AATATATTTTATATTATAAATTTATAAAAAATATTTAAATTTATATTATTATATAATTAAAATA
AATTAATAATTTAATTATTATTTTTTTTAATTTTTAATTTTTTATATAAATTTTAATTTTATAT
 
ATTATTAAAAGTTATATATTTTTAATTTATAAATAATTTATTTAATAACTTTTTAAGAATTCGG
TACC
SEQ ID NO: 5 - Primer rDNA7-FW
   GAATTCATTAAAGTTATTAATTATATATATATATATATATATATTAAAAG
SEQ ID NO: 6 - Primer rDNA7-REV
   GAATTCTTAAAAAGTTATTAAATAAATTATTTATAAATTAAAAATATATAAC

### B. Transformation

Spores of *Trichoderma reesei* strain Morph 1.1 pyr+ (see, *e.g*., U.S. Patent Publication Nos. US20090232788 and 20110136197) were transformed with 2 µg of the above-described pTrex3gM-AkAA plasmids harboring insulator DNA sequences (or a control plasmid without insulators) by means of biolistic transformation, and plated on amdS-selective plates. Stable transformants were transferred twice onto amdS selective agar plates.

### C. Fermentation and analysis of reporter protein expression

Stable transformants were used to inoculate glucose/sophorose defined medium [(NH₄)₂SO₄ 5 g/l; PIPPS buffer 33 g/l; Bacto Casamino Acids 9 g/l; KH₂PO₄ 4.5 g/l; CaCl₂ 1.3 g/l; MgSO₄.7H₂O 1 g/l; Mazu DF204 5 ml/l; 400X Trace Elements 2.5 ml/l; pH 5.5; glucose/sophorose (sterilized separately) 16 g/l. 400X Trace Elements solution: Citric acid (anhydrous) 175 g/l; FeSO₄.7H₂O 200 g/l; ZnSO₄.7H₂O 16 g/l; CuSO₄.5H₂O 3.2 g/l; MnSO₄.H₂O 1.4 g/l; H₃BO₃ 0.8 g/l.)]. Fermentation was performed at 28°C in the presence of oxygen and 100% humidity in 24-well plates without shaking. Samples were harvested after 5 days of fermentation and analyzed for amylase activity using the Ceralpha α-amylase kit (Megazyme), and for levels of protein expression using SDS-PAGE (4-10% Bis-Tris run in MOPS buffer). AkAA activity was determined of samples diluted in GSHE buffer (0.1 M NaOAc [pH 4.5], with 1% (w/v) of BSA added freshly before use). 40 µL sample was mixed with an equal volume of substrate and incubated at 28°C. After 10 min, the reaction was stopped by adding 100 µL stop solution (200 mM boric acid [pH 10.2]), and color absorption was read at 405 nm using a spectrophotometer (Spectramax).

Shake-flask scale fermentations were performed in 250-ml baffled shake flasks at 28°C and 250 rpm. 50 ml of YEG medium was inoculated and incubated for 2 days. 5 ml of fully grown YEG culture was used to inoculate 45 ml of NREL glucose/sophorose medium and incubated for 3-5 days. Samples were analyzed for α-amylase activity and levels of expression as described above.

### D. Effect of insulators on expression of secreted reporter protein

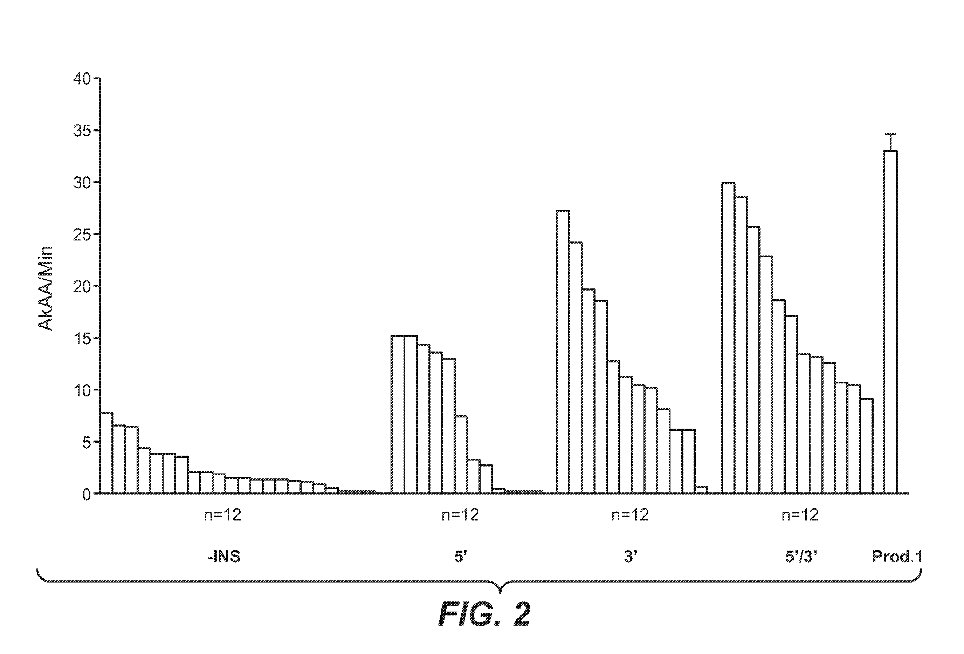

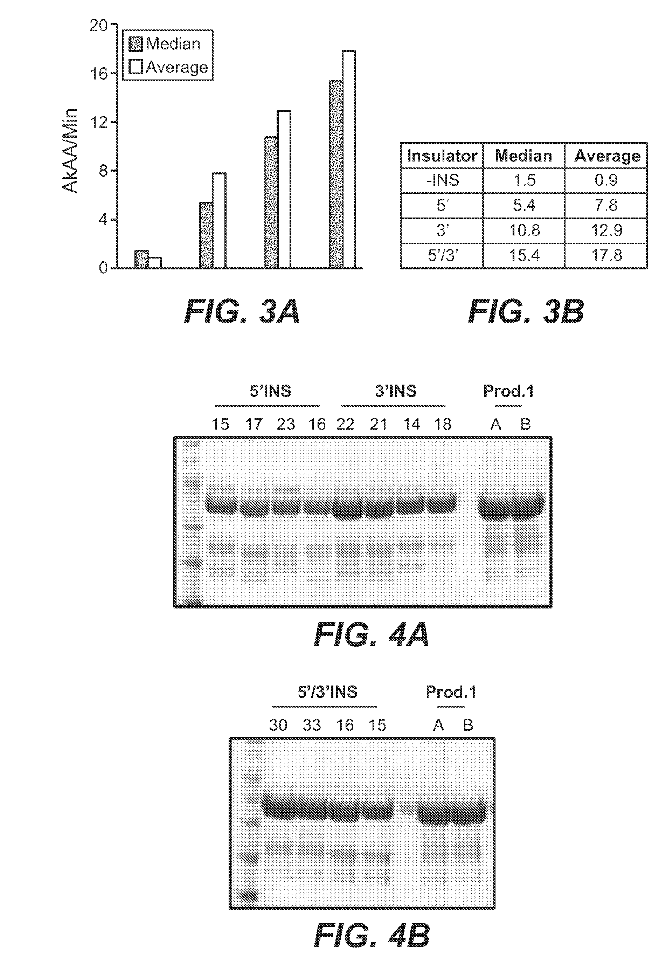

Figure 2 shows the effect of insulator DNA sequences on the expression of the reporter protein in transformants grown in 24-plates. Expression levels of 12 each (n=12) transformants, *i.e.*, no insulator (-ins), 5'-insertion) (5'), 3'-insertion (3'), or 5'and 3'-insertions (5'/3') were determined based on the amount of AkAA activity measured in the cell broth, and expressed as AkAA/min *(i.e.,* the increase in absorption at 405 nm per minute). An AkAA production strain (PROD1) was used as a reference. The production strain was previously selected based on the screening of a large number of transformants for maximum expression. The presence of the 5'-inserted insulator (5') significantly increased the expression levels of the transformants compared to the no-insulator control (-ins). The presence of the 3'-inserted insulator (3') had an even greater effect. The presence of both the 5' and 3'-inserted insulator (5'/3') had yet a greater effect, suggesting that the effect produced by the 5' and the 3' insulator was additive. The levels of AkAA production observed in only 12 transformants, approached those of the production strain (Prod1), which was obtained only after screening a large number of transformants. Figures 3A and 3B shows the mean and median expression levels produced by the top four transformants shown in Figure 2, further illustrating the significant increases in expression due to the presence of insulator sequences (clone numbers are indicated). The four highest expressing transformants shown in Figure 2 were also analyzed by SDS-PAGE (Figures 4A and 4B; the same clone numbers are indicated). The relative intensities of the AkAA bands compared to background bands are apparent, and show that the levels of AkAA, not the overall levels of protein, are increased as a result of the presence of insulators.

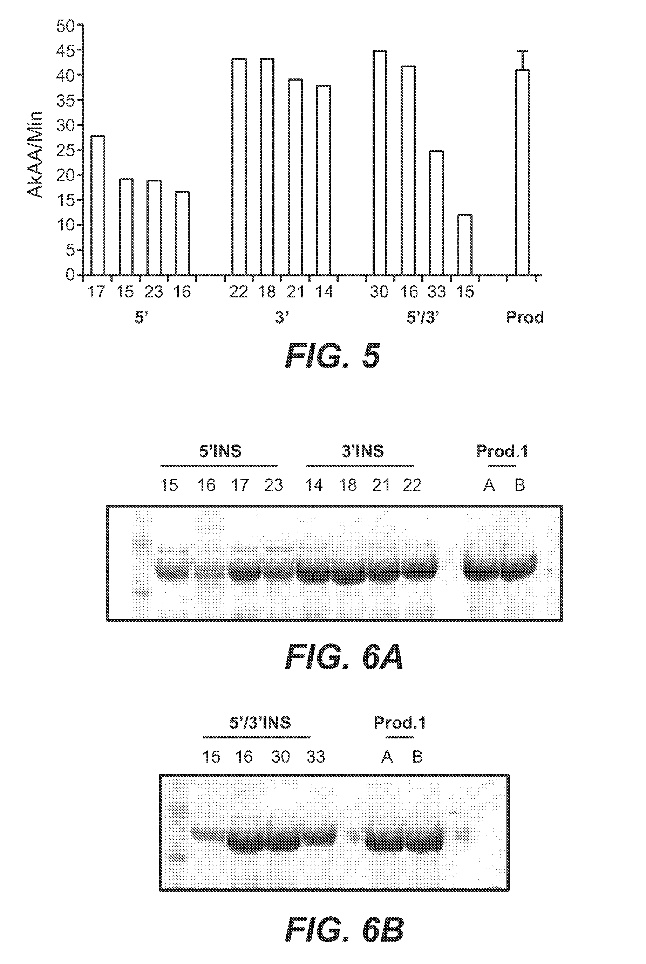

Figure 5 shows the effect of insulator DNA sequences on the expression of the reporter protein in transformants grown in shake flasks. The transformants are the same as those shown in Figures 3 and 4. The levels of reporter protein produced in shake flasks were generally higher, and the differences in expression levels less apparent and more compressed, than in the case of 24-well dishes. However, the presence of the insulators clearly improved expression. In some cases, the 3' and 5'/3' transformants appeared to express more reporter protein than the production strain (Prod). Figures 6A and 6B are Coomassie-stained SDS-PAGE gels showing the protein patterns produced by the transformants from Figure 5.

### E. Targeting insulated expression cassettes to a preselected locus

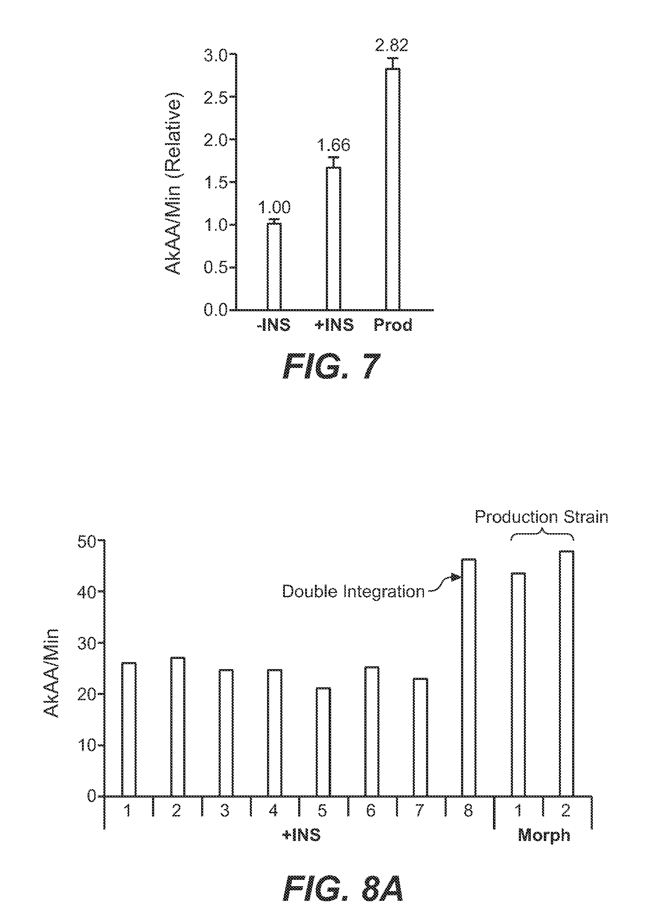

pTrex3gM-AkAA and the corresponding version of the plasmid with the 3'-inserted insulator (*i.e*., pTrex3gM-AkAA-3'INS) were digested by *Cla*I and used to transform the MAD6 strain of *Trichoderma reesei* (described in WO2010/141779) to obtain single-site, single copy stable transformants. MAD6 contains the *pyr2* gene reintroduced at the *pyr2* locus between a *T. reesei cbh1* promoter and the partial *amdS* selectable marker. MAD6 can be selected for uridine prototrophy and sensitivity to hygromycin. MAD6 transformants grown in 24-well plates were analyzed for expression levels of secreted AkAA as above. As shown in Figure 7, the presence of the 3' insulator increased AkAA expression levels from a single copy at the predetermined locus ~1.7 fold compared to transformants generated with pTrex3gM-AkAA. The error bars represent the standard deviations of five control (-INS) and seven 3'-insertion (+INS) transformants, respectively. The error bars for the production strain (Prod) represent the standard deviation of four independent inoculations the same production strain.

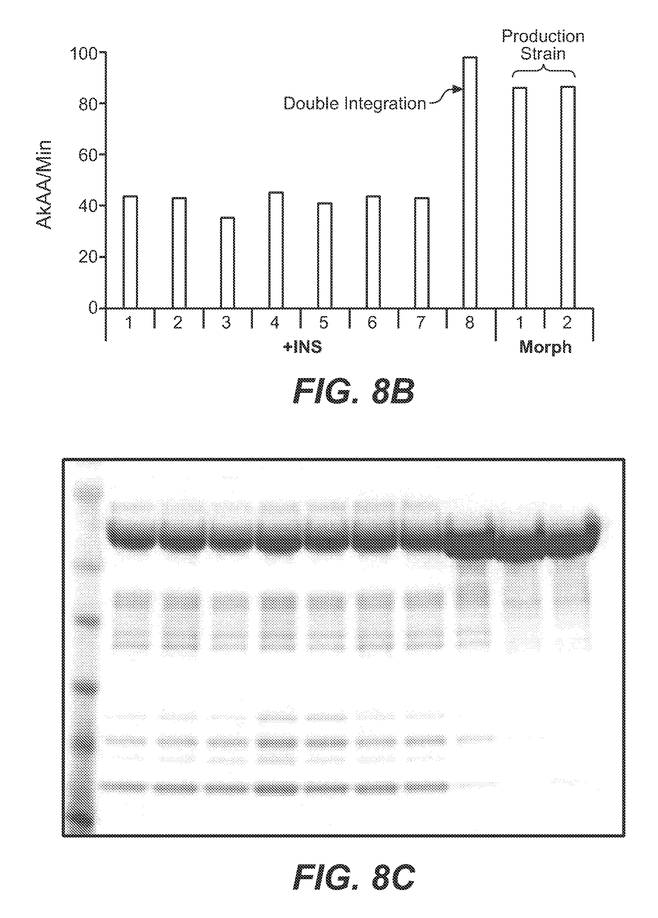

With a frequency of 10-20%, MAD6 transformants integrate two copies of a pTrex3gM expression cassette integrated at (presumably) the same locus. Indeed, a transformant was obtained that most likely contained two copies of the expression cassette pTrex3gM-AkAA-3'INS at the predetermined locus of MAD6 (Figures 8A-C, transformant and lane 8 compared to transformants and lanes 1-7). This transformant produces AkAA levels that equal those of the production strain (Morph). Figure 8A shows the results from 24-well plates. Figure 8B shows results from shake-flasks. Figure 8C shows the analysis of the transformants of Figure 8B by SDS-PAGE.

The results obtained in the MAD6 cells demonstrate that the insulated expression cassettes can be targeted to a preselected locus, and that only two insulated copies of a gene of interest are capable of expressing as much protein of interest as a production strain, which likely contains more than two copies of an expression cassette.

### F. Conclusions

Introducing an insulator into the expression cassette clearly had a significant effect on reporter protein expression levels. Maximum benefit was realized when the insulator was present both upstream and downstream of the expression cassette. Assuming that random integration events adjacent to insulator sequences are rare events, the ability to provide an insulator as part of an expression cassette offers significant advantages in terms of screening. In addition, the ability to introduce insulators both upstream and downstream of an expression cassette may allow even higher levels of expression than occur from random integration. The ability to target insulated expression cassettes to preselected loci reduces the chance of disrupting or activating other genes in the cell.

## Claims

1. An expression cassette for expressing a gene of interest in *Trichoderma* spp. cells, the expression cassette being comprised of a contiguous DNA sequence comprising:
a region encoding a gene of interest desired to be expressed;
one or more regulatory sequences sufficient to cause the expression of the gene of interest upon introduction of the expression vector into the *Trichoderma* spp. cells; and
an insulator sequence upstream and/or downstream of the contiguous DNA sequence, wherein the insulator sequence reduces histone binding;
wherein upon introduction of the expression cassette into the *Trichoderma* spp. cells, the presence of the insulator sequence increases the expression of the gene of interest compared to the level of expression obtained using an equivalent expression cassette lacking the insulator sequences.

2. The expression vector of claim 1, wherein:
(a) the one or more regulatory sequences includes a promoter, and the insulator sequence is placed upstream of the promoter; or
(b) the one or more regulatory sequences includes a terminator, and the insulator sequence is placed downstream of the terminator.

3. The expression vector of claim 1, wherein the one or more regulatory sequences include a promoter and a terminator, and a first insulator sequence is placed upstream of the promoter, and a second insulator sequence is placed downstream of the terminator.

4. The expression vector of any of claims 1-3, wherein the insulator sequence is a portion of an rRNA gene.

5. The expression vector of any of claims 1-4, wherein:
(a) the insulator sequence has a predicted histone occupancy probability of less than 0.3; or
(b) the insulator sequence has a predicted histone occupancy probability of less than 0.2.

6. The expression vector of any of claims 1-4, wherein the *Trichoderma* spp. is *Trichoderma reesei.*

7. An integration cassette for expressing a gene of interest in *Trichoderma* spp. cells, the integration cassette being comprised of a contiguous DNA sequence comprising:
a site of insertion for a gene of interest desired to be expressed; and
an insulator sequence upstream and/or downstream of the site of insertion, wherein the insulator sequence reduces histone binding;
wherein upon introduction of the integration cassette to *Trichoderma* spp. cells and upon introduction of the gene of interest at the site of insertion, the presence of the insulator sequence increases the expression of the gene of interest compared to the level of expression obtained using an equivalent expression cassette lacking the insulator sequences.

8. The integration cassette of claim 7, wherein:
(a) the integration cassette further comprises a promoter, and the insulator sequence is placed upstream of the promoter; or
(b) the integration cassette further comprises a terminator, and the insulator sequence is placed downstream of the terminator.

9. The integration cassette of claim 7, wherein the integration cassette further comprises a promoter and a terminator, and a first insulator sequence is placed upstream of the promoter, and a second insulator sequence is placed downstream of the terminator.

10. The integration cassette of any of claims 7-9, wherein the insulator sequence is a portion of an rRNA gene.

11. The integration cassette of any of claims 7-10, wherein:
(a) the insulator sequence has a predicted histone occupancy probability of less than 0.3; or the insulator sequence has a predicted histone occupancy probability of less than 0.2.

12. A method for expressing a gene of interest in *Trichoderma* spp. cells, the method comprising, introducing to the *Trichoderma* spp. cells
(i) a contiguous DNA sequence comprising a region encoding a gene of interest desired to be expressed and one or more regulatory sequences sufficient to cause the expression of the gene of interest in the *Trichoderma* spp. cells; and
(ii) an insulator sequence upstream or downstream of the contiguous DNA sequence in (i), wherein the insulator sequence reduces histone binding;
wherein the presence of the insulator sequence increases the expression of the gene of interest in the *Trichoderma* spp. cells compared to the level of expression obtained in the absence of the insulator sequences.

13. The method of claim 12, wherein:
(a) the contiguous DNA sequence and insulator sequence are introduced simultaneously into the *Trichoderma* spp. cells; or
(b) the contiguous DNA sequence and insulator sequence are introduced sequentially into the *Trichoderma* spp. cells.

14. The method of claim 12 or claim 13, wherein:
(a) the gene of interest and regulatory sequences are introduced simultaneously into the Trichoderma spp. cells as a contiguous DNA sequence; and/or
(b) the gene of interest and regulatory sequences are in an expression cassette; and/or
(c) the gene of interest and regulatory sequences are introduced sequentially into the Trichoderma spp. cells to form a contiguous DNA sequence.

15. The method of any of claims 12-14, wherein:
(a) the regulatory sequences comprises a promoter, and the insulator sequence is placed upstream of the promoter; and/or(b) the regulatory sequences comprises a terminator, and the insulator sequence is placed downstream of the terminator; and/or(c) the regulatory sequences comprise a promoter and a terminator, and a first insulator sequence is placed upstream of the promoter, and a second insulator sequence is placed downstream of the terminator.

16. The method of any of claims 12-15, wherein the insulator sequence is a portion of an rRNA gene.

17. The method of any of claims 12-16, wherein:
(a) the insulator sequence has a predicted histone occupancy probability of less than 0.3; or
(b)the insulator sequence has a predicted histone occupancy probability of less than 0.2.

18. The method of any of claims 12-17, wherein, wherein the *Trichoderma* spp. is *Trichoderma reesei.*

## Patentansprüche

1. Expressionskassette zum Exprimieren eines Gens von Interesse in *Trichoderma-*spp.-Zellen, wobei die Expressionskassette aus einer zusammenhängenden DNA-Sequenz besteht, die umfasst:
eine Region, codierend ein Gen von Interesse, von dem gewünscht wird, dass es exprimiert wird;
eine oder mehrere regulatorische Sequenzen, ausreichend, um bei Einführung des Expressionsvektors in die *Trichoderma*-spp.-Zellen die Expression des Gens von Interesse zu bewirken; und
eine Isolatorsequenz stromaufwärts und/oder stromabwärts von der zusammenhängenden DNA-Sequenz, wobei die Isolatorsequenz die Histon-Bindung verringert;
wobei bei Einführung der Expressionskassette in die *Trichoderma*-spp.-Zellen die Anwesenheit der Isolatorsequenz die Expression des Gens von Interesse, verglichen mit dem Niveau der Expression, erhalten unter Verwendung einer äquivalenten Expressionskassette, der die Isolatorsequenzen fehlen, erhöht.

2. Expressionsvektor nach Anspruch 1, wobei:
(a) die eine oder mehreren regulatorischen Sequenzen einen Promotor einschließen und die Isolatorsequenz stromaufwärts von dem Promotor angeordnet ist; oder
(b) die eine oder mehreren regulatorischen Sequenzen einen Terminator einschließen und die Isolatorsequenz stromabwärts von dem Terminator angeordnet ist.

3. Expressionsvektor nach Anspruch 1, wobei die eine oder mehreren regulatorischen Sequenzen einen Promotor und einen Terminator einschließen und eine erste Isolatorsequenz stromaufwärts von dem Promotor angeordnet ist und eine zweite Isolatorsequenz stromabwärts von dem Terminator angeordnet ist.

4. Expressionsvektor nach einem der Ansprüche 1-3, wobei die Isolatorsequenz ein Anteil von einem rRNA-Gen ist.

5. Expressionsvektor nach einem der Ansprüche 1-4, wobei:
(a) die Isolatorsequenz eine vorhergesagte Histon-Besetzungswahrscheinlichkeit von weniger als 0,3 hat; oder
(b) die Isolatorsequenz eine vorhergesagte Histon-Besetzungswahrscheinlichkeit von weniger als 0,2 hat.

6. Expressionsvektor nach einem der Ansprüche 1-4, wobei die *Trichoderma* spp. *Trichoderma reesei* ist.

7. Integrationskassette zum Exprimieren eines Gens von Interesse in *Trichoderma-*spp.-Zellen, wobei die Integrationskassette aus einer zusammenhängenden DNA-Sequenz besteht, die umfasst:
eine Insertionsstelle für ein Gen von Interesse, von dem gewünscht wird, dass es exprimiert wird; und
eine Isolatorsequenz stromaufwärts und/oder stromabwärts von der Insertionsstelle, wobei die Isolatorsequenz die Histon-Bindung verringert;
wobei bei Einführung der Integrationskassette in *Trichoderma*-spp.-Zellen und bei Einführung des Gens von Interesse in die Insertionsstelle die Anwesenheit der Isolatorsequenz die Expression des Gens von Interesse, verglichen mit dem Niveau der Expression, erhalten unter Verwendung einer äquivalenten Expressionskassette, der die Isolatorsequenzen fehlen, erhöht.

8. Integrationskassette nach Anspruch 7, wobei:
(a) die Integrationskassette weiterhin einen Promotor umfasst und die Isolatorsequenz stromaufwärts von dem Promotor angeordnet ist; oder
(b) die Integrationskassette weiterhin einen Terminator umfasst und die Isolatorsequenz stromabwärts von dem Terminator angeordnet ist.

9. Integrationskassette nach Anspruch 7, wobei die Integrationskassette weiterhin einen Promotor und einen Terminator umfasst und eine erste Isolatorsequenz stromaufwärts von dem Promotor angeordnet ist und eine zweite Isolatorsequenz stromabwärts von dem Terminator angeordnet ist.

10. Integrationskassette nach einem der Ansprüche 7-9, wobei die Isolatorsequenz ein Anteil von einem rRNA-Gen ist.

11. Integrationskassette nach einem der Ansprüche 7-10, wobei:
(a) die Isolatorsequenz eine vorhergesagte Histon-Besetzungswahrscheinlichkeit von weniger als 0,3 hat; oder
(b) die Isolatorsequenz eine vorhergesagte Histon-Besetzungswahrscheinlichkeit von weniger als 0,2 hat.

12. Verfahren zum Exprimieren eines Gens von Interesse in *Trichoderma*-spp.-Zellen, wobei das Verfahren umfasst, in die *Trichoderma*-spp.-Zellen
(i) eine zusammenhängende DNA-Sequenz, umfassend eine Region, codierend ein Gen von Interesse, von dem gewünscht wird, dass es exprimiert wird, und eine oder mehrere regulatorische Sequenzen, ausreichend, um die Expression des Gens von Interesse in den *Trichoderma*-spp.-Zellen zu bewirken; und
(ii) eine Isolatorsequenz stromaufwärts oder stromabwärts von der zusammenhängenden DNA-Sequenz in (i), wobei die Isolatorsequenz die Histon-Bindung verringert;
einzuführen, wobei die Anwesenheit der Isolatorsequenz die Expression des Gens von Interesse in den *Trichoderma*-spp.-Zellen, verglichen mit dem Niveau der Expression, erhalten in Abwesenheit der Isolatorsequenzen, erhöht.

13. Verfahren nach Anspruch 12, wobei:
(a) die zusammenhängende DNA-Sequenz und die Isolatorsequenz gleichzeitig in die *Trichoderma*-spp.-Zellen eingeführt werden; oder
(b) die zusammenhängende DNA-Sequenz und die Isolatorsequenz nacheinander in die *Trichoderma*-spp.-Zellen eingeführt werden.

14. Verfahren nach Anspruch 12 oder Anspruch 13, wobei:
(a) das Gen von Interesse und die regulatorischen Sequenzen gleichzeitig in die *Trichoderma*-spp.-Zellen als eine zusammenhängende DNA-Sequenz eingeführt werden; und/oder
(b) das Gen von Interesse und die regulatorischen Sequenzen in einer Expressionskassette sind; und/oder
(c) das Gen von Interesse und die regulatorischen Sequenzen nacheinander in die *Trichoderma*-spp.-Zellen eingeführt werden, um eine zusammenhängende DNA-Sequenz zu bilden.

15. Verfahren nach einem der Ansprüche 12-14, wobei:
(a) die regulatorischen Sequenzen einen Promotor umfassen und die Isolatorsequenz stromaufwärts von dem Promotor angeordnet ist; und/oder
(b) die regulatorischen Sequenzen einen Terminator umfassen und die Isolatorsequenz stromabwärts von dem Terminator angeordnet ist; und/oder
(c) die regulatorischen Sequenzen einen Promotor und einen Terminator umfassen, und eine erste Isolatorsequenz stromaufwärts von dem Promotor angeordnet ist, und eine zweite Isolatorsequenz stromabwärts von dem Terminator angeordnet ist.

16. Verfahren nach einem der Ansprüche 12-15, wobei die Isolatorsequenz ein Anteil von einem rRNA-Gen ist.

17. Verfahren nach einem der Ansprüche 12-16, wobei:
(a) die Isolatorsequenz eine vorhergesagte Histon-Besetzungswahrscheinlichkeit von weniger als 0,3 hat; oder
(b) die Isolatorsequenz eine vorhergesagte Histon-Besetzungswahrscheinlichkeit von weniger als 0,2 hat.

18. Verfahren nach einem der Ansprüche 12-17, wobei die *Trichoderma* spp. *Trichoderma reesei* ist.

## Revendications

1. Cassette d'expression pour exprimer un gène d'intérêt dans des cellules de *Trichoderma* spp., la cassette d'expression étant constituée d'une séquence d'ADN contiguë comprenant:
une région codant un gène d'intérêt que l'on désire exprimer;
une ou plusieurs séquences régulatrices suffisante pour causer l'expression du gène d'intérêt lors de l'introduction du vecteur d'expression dans les cellules de *Trichoderma* spp.; et
une séquence isolatrice en amont et/ou en aval de la séquence d'ADN contiguë, où la séquence isolatrice réduit la liaison de l'histone;
où lors de l'introduction de la cassette d'expression dans les cellules de *Trichoderma* spp., la présence de la séquence isolatrice augmente l'expression du gène d'intérêt par comparaison au niveau d'expression obtenu en utilisant une cassette d'expression équivalente dépourvue de séquences isolatrices.

2. Vecteur d'expression selon la revendication 1, dans lequel:
(a) l'une ou les plusieurs séquences régulatrices comprennent un promoteur, et la séquence isolatrice est placée en amont du promoteur; ou bien
(b) l'une ou les plusieurs séquences régulatrices comprennent un terminateur, et la séquence isolatrice est placée en aval du terminateur.

3. Vecteur d'expression selon la revendication 1, dans lequel l'une ou les plusieurs séquences régulatrices comprennent un promoteur et un terminateur, et une première séquence isolatrice est placée en amont du promoteur, et une deuxième séquence isolatrice est placée en aval du terminateur.

4. Vecteur d'expression selon l'une quelconque des revendications 1-3, dans lequel la séquence isolatrice est une partie d'un gène d'ARNr.

5. Vecteur d'expression selon l'une quelconque des revendications 1-4, dans lequel:
(a) la séquence isolatrice a une probabilité prédite d'occupation par l'histone de moins de 0,3; ou bien
(b) la séquence isolatrice a une probabilité prédite d'occupation par l'histone de moins de 0,2.

6. Vecteur d'expression selon l'une quelconque des revendications 1-4, dans lequel la *Trichoderma* spp. est *Trichoderma reesei.*

7. Cassette d'intégration pour exprimer un gène d'intérêt dans des cellules de *Trichoderma* spp., la cassette d'intégration étant constituée d'une séquence d'ADN contiguë comprenant:
un site d'insertion pour un gène d'intérêt que l'on désire exprimer; et
une séquence isolatrice en amont et/ou en aval du site d'insertion, où la séquence isolatrice réduit la liaison de l'histone;
où lors de l'introduction de la cassette d'intégration dans des cellules de *Trichoderma* spp. et lors de l'introduction du gène d'intérêt au site d'insertion, la présence de la séquence isolatrice augmente l'expression du gène d'intérêt par comparaison au niveau d'expression obtenu en utilisant une cassette d'expression équivalente dépourvue des séquences isolatrices.

8. Cassette d'intégration selon la revendication 7, dans laquelle:
(a) la cassette d'intégration comprend en outre un promoteur, et la séquence isolatrice est placée en amont du promoteur, ou bien
(b) la cassette d'intégration comprend en outre un terminateur, et la séquence isolatrice est placée en aval du terminateur.

9. Cassette d'intégration selon la revendication 7, où la cassette d'intégration comprend en outre un promoteur et un terminateur, et une première séquence isolatrice est placée en amont du promoteur, et une deuxième séquence isolatrice est placée en aval du terminateur.

10. Cassette d'intégration selon l'une quelconque des revendications 7-9, dans laquelle la séquence isolatrice est une partie d'un gène d'ARNr.

11. Cassette d'intégration selon l'une quelconque des revendications 7-10, dans laquelle:
(a) la séquence isolatrice a une probabilité prédite d'occupation par l'histone de moins de 0,3; ou bien
(b) la séquence isolatrice a une probabilité prédite d'occupation par l'histone de moins de 0,2.

12. Procédé d'expression d'un gène d'intérêt dans des cellules de *Trichoderma* spp., le procédé comprenant introduire dans les cellules de *Trichoderma* spp.:
(i) une séquence d'ADN contiguë comprenant une région codant un gène d'intérêt que l'on désire exprimer et une ou plusieurs séquences régulatrices suffisantes pour causer l'expression du gène d'intérêt dans les cellules de *Trichoderma* spp.; et
(ii) une séquence isolatrice en amont ou en aval de la séquence d'ADN contiguë dans (i), où la séquence isolatrice réduit la liaison de l'histone;
dans lequel la présence de la séquence isolatrice augmente l'expression du gène d'intérêt dans les cellules de *Trichoderma* spp., par comparaison au niveau d'expression obtenu en l'absence des séquences isolatrices.

13. Procédé selon la revendication 12, dans lequel:
(a) la séquence d'ADN contiguë et la séquence isolatrice sont introduites simultanément dans les cellules de *Trichoderma* spp.; ou bien
(b) la séquence d'ADN contiguë et la séquence isolatrice sont introduites séquentiellement dans les cellules de *Trichoderma* spp.

14. Procédé selon la revendication 12 ou la revendication 13, dans lequel:
(a) le gène d'intérêt et les séquences régulatrices sont introduits simultanément dans les cellules de *Trichoderma* spp. comme une séquence d'ADN contiguë; et/ou
(b) le gène d'intérêt et les séquences régulatrices sont dans une cassette d'expression; et/ou
(c) le gène d'intérêt et les séquences régulatrices sont introduits séquentiellement dans les cellules de *Trichoderma* spp. pour former une séquence d'ADN contiguë.

15. Procédé selon l'une quelconque des revendications 12-14, dans lequel:
(a) les séquences régulatrices comprennent un promoteur, et la séquence isolatrice est placée en amont du promoteur; et/ou
(b) les séquences régulatrices comprennent un terminateur, et la séquence isolatrice est placée en aval du terminateur; et/ou
(c) les séquences régulatrices comprennent un promoteur et un terminateur, et une première séquence isolatrice est placée en amont du promoteur, et une deuxième séquence isolatrice est placée en aval du terminateur.

16. Procédé selon l'une quelconque des revendications 12-15, dans lequel la séquence isolatrice est une partie d'un gène d'ARNr.

17. Procédé selon l'une quelconque des revendications 12-16, dans lequel:
(a) la séquence isolatrice a une probabilité prédite d'occupation par l'histone de moins de 0,3; ou bien
(b) la séquence isolatrice a une probabilité prédite d'occupation par l'histone de moins de 0,2.

18. Procédé selon l'une quelconque des revendications 12-17, dans lequel la *Trichoderma* spp. est *Trichoderma reesei.*
